(19) Europäisches Patentamt

European Patent Office

Office européen des brevets

(11) **EP 2 164 540 B1**

(12) **EUROPÄISCHE PATENTSCHRIFT**

(45) Veröffentlichungstag und Bekanntmachung des Hinweises auf die Patenterteilung:
**08.08.2012 Patentblatt 2012/32**

(21) Anmeldenummer: **08802931.9**

(22) Anmeldetag: **18.06.2008**

(51) Int Cl.:
*A61M 1/00* *(2006.01)*

(86) Internationale Anmeldenummer:
**PCT/EP2008/057717**

(87) Internationale Veröffentlichungsnummer:
**WO 2009/007212 (15.01.2009 Gazette 2009/03)**

(54) **Vorrichtung zur Reduzierung von Druckschwankungen in einem Aspirationszweig und chirurgisches System**

Device for reducing pressure variations in an aspiration branch, and surgical system

Dispositif de réduction des variations de pression dans une branche d'aspiration, et système chirurgical

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **06.07.2007 DE 102007031722**

(43) Veröffentlichungstag der Anmeldung:
**24.03.2010 Patentblatt 2010/12**

(73) Patentinhaber: **Carl Zeiss Meditec AG**
**07745 Jena (DE)**

(72) Erfinder:
• **KÜBLER, Christoph**
**73447 Oberkochen (DE)**
• **KRAUS, Martin**
**73460 Hüttlingen (DE)**

• **EICHLER, Michael**
**Aalen**
**73434 (DE)**
• **MAIER, Tobias**
**73430 Aalen (DE)**

(74) Vertreter: **Lechner, Armin Anton**
**Hofstetter, Schurack & Skora**
**Patentanwälte**
**Balanstraße 57**
**81541 München (DE)**

(56) Entgegenhaltungen:
**WO-A-89/03230          DE-C1- 3 501 655**
**US-A- 4 182 385          US-A- 5 441 482**
**US-B1- 6 599 271**

**Beschreibung**

Vorrichtung zur Reduzierung von Druckschwankungen in einem Aspirationszweig und chirurgisches System

**Technisches Gebiet**

Die Erfindung betrifft eine Vorrichtung zur Reduzierung von Druckschwankungen eines in einem Aspirationszweig für ein chirurgisches System strömenden Fluids sowie ein chirurgisches System, insbesondere ein ophthalmisches mikrochirurgisches System zur Phako-Chirurgie.

**Stand der Technik**

Eine sehr häufig verwendete Methode in der Augenheilkunde ist die Phako-Emulsifizierung, bei welcher ein chirurgisches Handstück als mikrochirurgisches Werkzeug verwendet wird. Dieses Handstück umfasst im Allgemeinen eine Spitze in Form einer Hohlnadel mit einem relativ geringen Durchmesser, die zum Emulsifizieren, Fragmentieren und/oder Schneiden von Gewebe ausgelegt sein kann, nachdem diese in einen Einschnitt in der Cornea oder Sklera des Auges eingeführt worden ist. Zusätzlich kann diese Spitze des Handstücks einen zentralen Kanal aufweisen, der mit einer Saugquelle, beispielsweise einer Pumpe, verbunden ist, welche die Gewebereste der zertrümmerten Linse von dem Auge absaugt. Das Handstück kann des Weiteren zum Zuführen eines Spülfluids, beispielsweise eine Salzlösung (BSS-Lösung), zum Auge zum Spülen des Auges während der Behandlung ausgebildet sein. Das entfernte Gewebe, das von dem Auge zusammen mit dem Spülfluid abgesaugt wird, wird beispielsweise in einem Sammelbehälter gesammelt, welcher üblicherweise entfernt von dem Handstück angeordnet ist. Das Handstück umfasst zum Zertrümmern der Linse des Auges typischerweise eine Ultraschalleinrichtung, welche die Spitze des Auges zur Oszillation anregt. Durch diese Oszillation der Spitze wird die Linse in kleine Teile zertrümmert.

Das Fluidiksystem bei einem Phako-Emulsifikationssystem gliedert sich in zwei Funktionsgruppen. Das Irrigationssystem führt während der Operation die Bewässerung des Auges mit einem Spülfluid durch. Simultan wird über ein Pumpenaspirationssystem das durch den Ultraschallprozess emulsifizierte Linsenmaterial abgesaugt. Das Fluidikmodul ist mit diesen beiden Funktionen über ein flexibles Schlauchsystem mit dem Phako-Handstück verbunden. Die Zuführung des Spülfluids während der Operationsphase wird im Irrigationszweig über ein Irrigationsventil gesteuert. Während des Absaugens wird der Saugdruck im Aspirationszweig gemessen und für die Überwachung und Steuerung der Fluidik- und Ultraschallsysteme eingesetzt.

Bei diesen chirurgischen Systemen kann es im Betrieb der Komponenten zu Druckschwankungen des Fluids kommen. Insbesondere kann dies im Aspirationszweig auftreten, wodurch das Absaugen lediglich suboptimal erfolgen kann und den Operationsablauf beeinträchtigen kann.

Aus der US 4,182,385 und der WO 89/03230 sind chirurgische Systeme zur Phakochirurgie bekannt. In einem Aspirationszweig ist dabei eine Pumpe zur Förderung eines Fluids angeordnet.

**Darstellung der Erfindung**

Es ist Aufgabe der vorliegenden Erfindung, eine Vorrichtung sowie ein chirurgisches System zu schaffen, mit welcher bzw. mit welchem das Frequenzspektrum bzw. das Wellenlängen-/Amplitudenspektrum dieser Druckpulsationen gedämpft werden kann.

Diese Aufgabe wird durch eine Vorrichtung sowie ein chirurgisches System gemäß den unabhängigen Ansprüchen gelöst.

Eine erfindungsgemäße Vorrichtung ist zur Reduzierung von Druckschwankungen eines in einem Aspirationszweig für ein chirurgisches System strömenden Fluids ausgebildet. Die Vorrichtung umfasst eine Pumpe, welche dem Aspirationszweig zugeordnet und insbesondere mit diesem verbunden ist, und welche im aktiven Betrieb zur nichtkontinuierlichen Förderung des strömenden Fluids ausgebildet ist. Durch diese Pumpe und ihre Betriebsart werden im Aspirationszweig Druckschwankungen des Fluids generiert. Die Vorrichtung umfasst des Weiteren eine Diffusoranordnung, welche im Aspirationszweig in Strömungsrichtung des Fluids vor der Pumpe angeordnet ist. Durch diese Vorrichtung kann das Frequenzspektrum bzw. das Wellenlängen-/Amplitudenspektrum dieser Druckpulsationen so verändert werden, dass auch die Dämpfungswirkung im Aspirationszweig wesentlich verbessert ist.

Im nachfolgenden Kontext wird anstelle des Begriffs Reduzierung von Druckschwankungen die Formulierung Dämpfung von Druckschwankungen verwendet, wobei stets eine Reduzierung im Sinne einer Verstimmung einer Übertragungscharakteristik des Systems verstanden werden soll und nicht eine Energievernichtung im Sinne einer klassischen Dämpfung durch Energieumwandlung.

Die Diffusoranordnung weist ein Verhältnis K zwischen ihrem maximalen Innenmaß und einem minimalen Innenmaß auf, welches größer oder gleich 2 ist. Insbesondere bei einem runden Querschnitt ist das Innenmaß als Innendurchmesser ausgebildet. Bevorzugt weist die Diffusoranordnung ein Verhältnis K zwischen ihrem maximalen Innenmaß und ihrem minimalen Innenmaß auf, welches größer oder gleich 5, insbesondere größer oder gleich 10, ist. Bereits mit relativ kleinen Werte von K lassen sich wesentliche Dämpfungen insbesondere der Amplitude der erzeugten Druckwelle erreichen. Neben der vorrangigen Dämpfung der Amplitude der Welle wird auch die Phasengeschwindigkeit bzw. Wellenausbreitungsgeschwindigkeit c dadurch variiert.

Bevorzugterweise wird im Aspirationszweig bzw. auf der Aspirationsseite eines chirurgischen Systems eine Saugpumpe mit geschlossener Bauweise verwendet. Dies bedeutet, dass die Pumpe eine Bauart aufweist, bei der die das Fluid fördernden bzw. weitertransportierenden Elemente der Pumpe nicht unmittelbar mit dem Fluid in Kontakt sind. Insbesondere kann als Pumpe eine Peristaltikpumpe eingesetzt werden. Ebenso können jedoch auch Pumpen der Bauart Scrollpumpe oder Diaphragmapumpe eingesetzt werden. Gerade diese Pumpentypen sind durch ihr Bauartprinzip dadurch gekennzeichnet, dass sie im aktiven Betrieb keinen kontinuierlichen Fluidstrom generieren können, da die Öffnungs- bzw. Schließventile oder die Rollenräder jeweils eine abgeschlossene Volumenkammer aus dem Saugbereich entziehen. Aus diesem Grund induzieren diese Pumpen im Aspirationszweig des Operationsgeräts durch ihre Strömungspulse auch Druckpulsationen des strömenden Fluids.

Da gerade derartige Pumpen, welche mit ihren Förderelementen nicht unmittelbar in Kontakt mit dem strömenden Fluid sind, nicht zuletzt aus hygienischen Gründen im chirurgischen Bereich bevorzugt eingesetzt werden, ist es insbesondere bei solchen Ausgestaltungen erforderlich, die damit einhergehenden Druckschwankungen bei der nichtkontinuierlichen Förderung des Fluids durch die Pumpe zu dämpfen. Nicht zuletzt ist dies deshalb erforderlich, um das Operationsergebnis nicht negativ zu beeinträchtigen. Bevorzugt umfasst die Diffusoranordnung zumindest einen Diffusor. Prinzipiell werden mit der Bezeichnung Diffusor auch alle diejenigen Elemente verstanden, welche die Geschwindigkeit der Wellenausbreitung bei derartigen Druckschwankungen des strömenden Fluids reduzieren, insbesondere deutlich reduzieren können. Besonders soll jedoch die Amplitude der Druckwelle mit dem Diffusor reduziert werden.

Bevorzugt ist vorgesehen, dass die Diffusoranordnung zumindest zwei Diffusoren umfasst. Diese Diffusoren können als separate Elemente ausgebildet sein. Insbesondere kann vorgesehen sein, dass bei einer Mehrzahl von Diffusoren diese beabstandet zueinander im Aspirationszweig angeordnet sind. Ebenso kann jedoch auch vorgesehen sein, dass die Diffusoren unmittelbar aneinander angrenzend angeordnet sind. Situationsabhängig kann dann eine optimierte Anzahl und eine optimierte Anordnung der Diffusoren zueinander ausgebildet werden.

Bei einer Mehrzahl von Diffusoren kann vorgesehen sein, dass diese gleich ausgebildet sind. Insbesondere in Formgebung und den Ausmaßen sowie in der Materialwahl können diese gleich ausgebildet sein.

Ebenso kann jedoch auch vorgesehen sein, dass zumindest zwei Diffusoren unterschiedlich ausgebildet sind. Insbesondere kann auch hier in zumindest einem der Parameter Materialwahl, Formgebung und Ausmaßen eine Unterschiedlichkeit vorliegen.

Umfasst die Diffusoranordnung zumindest zwei Diffusoren, so kann vorgesehen sein, dass diese parallel zueinander geschaltet bzw. angeordnet sind. Ebenso kann jedoch auch vorgesehen sein, dass zumindest zwei Diffusoren in Serie zueinander geschaltet sind. Durch die Serienschaltung oder die Parallelschaltung der Diffusoren kann auch hier situationsspezifisch eine optimale Dämpfung von Druckschwankungen erreicht werden, da abhängig von den sonstigen Systemparametern im Aspirationszweig und weiteren Komponenten des chirurgischen Systems das Frequenzspektrum der Druckpulsationen des strömenden Fluids bzw. der Wellenausbreitung unterschiedlich sein kann. Individuell kann dann durch die Anordnung der Diffusoren sehr exakt und genau die Dämpfung spezifischer Frequenzbereiche erreicht werden.

Bei einer Mehrzahl von Diffusoren können diese lösbar miteinander verbunden sein. In einfacher und aufwandsarmer Weise kann dann eine quasi beliebige Anzahl von Diffusoren zusammengesetzt werden, um eine spezifische Diffusoranordnung erhalten zu können. Dadurch kann ein modularer Aufbau der Diffusoranordnung ermöglicht werden.

Darüber hinaus kann der Austausch oder die Wartung eines einzelnen Diffusors aufwandsarm erfolgen. Eine derartig zerstörungsfrei lösbare und reversibel erzeugbare Verbindung kann auch das Durchführen von Reinigungsarbeiten oder dergleichen vereinfachen.

Ebenso kann jedoch auch vorgesehen sein, dass zumindest zwei Diffusoren unlösbar miteinander verbunden sind. Eine derartige integrale Ausbildung der Diffusoren ermöglicht ein kostengünstiges und einfaches Herstellen der Elemente. Ebenso können dadurch Toleranzen an den Schnittstellen der einzelnen Diffusoren der Gesamtanordnung bei relativ komplexer Ausgestaltung minimiert werden. Auch die mechanische Stabilität kann dadurch positiv beeinflusst werden.

Bevorzugt ist die Diffusoranordnung in einer Aspirationsleitung oder einem Aspirationsschlauch des Aspirationszweigs angeordnet. Dadurch kann auch die Dämpfungswirkung einer Aspirationsleitung oder des Aspirationsschlauchs, welche bzw. welcher vorzugsweise zumindest bereichsweise durch die Druckschwankungen des strömenden Fluids elastisch verformbar sein können, besser ausgenutzt werden.

Bevorzugterweise ist die Diffusoranordnung beabstandet zur im Aspirationszweig angeordneten Pumpe und beabstandet zu einem mit einem elastischen Aspirationsschlauch des Aspirationszweigs verbundenen chirurgischen Handstück, insbesondere einem Phako-Handstück, zwischen der Pumpe und dem Handstück angeordnet. Vorzugsweise stellt der Aspirationsschlauch bei dieser Ausführung die Verbindung zwischen dem Handstück und der Diffusranordnung dar.

Es kann vorgesehen sein, dass die Diffusoranordnung zerstörungsfrei lösbar in dem Aspirationszweig angeordnet ist. Auch dadurch kann somit ein einfaches reversibles Austauschen und Wiedereinsetzen oder ein Ersetzen der Diffusoranordnung bei einem Defekt oder zum Reinigen ermöglicht werden.

Es kann jedoch auch vorgesehen sein, dass die Diffusoranordnung integral und somit unlösbar in dem Aspirationszweig ausgebildet ist. Durch diese Ausgestaltung kann quasi ein einstückiges Element bereitgestellt werden, welches als Ganzes schnell in das System eingesetzt oder aus diesem entnommen werden kann. Darüber hinaus können auch kritische Stellen beispielsweise im Hinblick auf Dichtigkeit oder dergleichen durch eine derartige integrale Ausbildung verhindert werden. Darüber hinaus kann auch die Fertigung kostengünstig realisiert werden. Beispielsweise kann diesbezüglich ein Spritzgussteil vorgesehen sein.

Bevorzugterweise ist ein minimales Innenmaß, insbesondere ein minimaler Innendurchmesser, des mit Fluid durchströmten Bereichs eines Diffusors, insbesondere die Strömungskanalanordnung des Diffusors, größer oder gleich einem minimalen Innenmaß, insbesondere Innendurchmesser, einer vorderen Öffnung einer Hohlnadel des Handstücks. Durch diese Dimensionierung kann verhindert werden, dass in die Hohlnadel eingesaugte Linsenreste in der Diffusoranordnung hängen bleiben und zu einer Verstopfung führen.

Bevorzugterweise ist das minimale Innenmaß, insbesondere der Innendurchmesser, der Diffusoranordnung zwischen 0,8 mm und 2,5 mm, insbesondere zwischen 0,8 mm und 1,5 mm.

Ein maximales Innenmaß, insbesondere Innendurchmesser, des mit Fluid durchströmten Bereichs der Diffusoranordnung und somit insbesondere des Strömungskanals der Diffusoranordnung, beträgt vorzugsweise zwischen 1,2 mm und 25 mm, insbesondere zwischen 1,5 mm und 15 mm. Durch diese Dimensionierungen der minimalen und maximalen Innenmaße kann eine optimale Dämpfung der Druckpulsationen des strömenden Fluids erreicht werden.

Die Länge der Diffusoranordnung kann abhängig von spezifischen Parametern des Fluids und/oder von dem den Aspirationszweig zugeordneten Komponenten sein. Insbesondere ist die Länge der Diffusoranordnung abhängig von der Ausgestaltung der Leitungen und/oder Schläuche des Aspirationszweigs und/oder der Pumpe und/oder der Diffusoranordnung selbst. Beispielsweise können hierbei die Form, die Ausmaße, das Material und der Anbringungsort und die Anzahl der Diffusoren berücksichtigt werden.

Vorzugsweise ist die Diffusoranordnung mit einem elastischen Aspirationsschlauch im Aspirationszweig verbunden und die Länge der Diffusoranordnung ist kleiner 15% der Länge des Schlauchs. Bevorzugt ist die Länge der Diffusoranordnung kleiner oder gleich 5%, insbesondere kleiner oder gleich 2% der Länge des Schlauchs. Je kleiner die Länge der Diffusoranordnung im Vergleich zu anderen Komponenten ausgebildet ist, um so einfacher und flexibler ist die Handhabbarkeit und die Verwendbarkeit der Anordnung. Insbesondere kann dadurch auch ein kompakter und bauraumminimierter Aufbau erreicht werden. Nicht zuletzt kann dadurch auch Gewicht gespart werden, was insbesondere bei einer nahen Anbringung der Diffusoranordnung zum Handstück, welches vom Operateur gehalten und bewegt werden muss, zu einer wesentlich verbesserten Bedienbarkeit und leichten Verwendbarkeit führt.

Vorzugsweise weist die Diffusoranordnung eine Länge zwischen 10 mm und 400 mm, insbesondere zwischen 90 mm und 300 mm, vorzugsweise kleiner oder gleich 200 mm, auf. Diese Längenbereiche ermöglichen eine besonders geeignete Ausgestaltung im Hinblick auf Bauraumminimierung und effektiver Dämpfung der Druckpulsationen. Die Länge der Diffusoranordnung kann jedoch auch zwischen 100 mm und 400 mm oder zwischen 20 mm und 200 mm liegen. Systemabhängig kann dann die jeweils optimale Geometrie gewählt werden.

Als spezifische Parameter, von welchen vorteilhafterweise die Länge und Geometrie der Diffusoranordnung abhängig ausgebildet wird, kann beispielsweise die Wellenfrequenz der Druckpulsation des strömenden Fluids und/oder die Dichte des Fluids und/oder die Wanddicke der Diffusoranordnung bzw. eines Diffusor und/oder das Young-Modul E der Wandung des Diffusors berücksichtigt werden. Anhand dieser Parameter und zusätzlicher Größen, wie beispielsweise dem lokalen Durchmesser des Diffusors und/oder der dynamischen Viskosität von Wasser kann die Wellenausbreitung in einer dreidimensionalen Simulation ermittelt werden und daraus dann eine geeignete Geometrie der Diffusoranordnung bestimmt werden. Bevorzugt wird dabei ein Verhältnis K zwischen einem maximalen und einem minimalen Innenmaß der Diffusoranordnung bestimmt und für die Optimierung als Variable in der Simulation verändert. Insbesondere die Größe K ist der wesentliche Einflussfaktor im Hinblick auf eine Dämpfung der Druckwelle im Fluid.

Es kann vorgesehen sein, dass eine Innenseite der Diffusoranordnung, welche eine Begrenzung des von Fluid durchströmten Bereichs der Diffusoranordnung darstellt und somit insbesondere eine Wand eines Strömungskanals definiert, zumindest bereichsweise für die durch Druckpulse bzw. Druckwellen des Fluids verursachten Krafteinwirkungen elastisch ist. Ebenso kann jedoch auch vorgesehen sein, dass eine derartige Innenseite der Diffusoranordnung zumindest bereichsweise für eine von dem Fluid verursachte Krafteinwirkung starr und somit unverformbar ist.

Die Diffusoranordnung ist vorzugsweise in einer Fluidik-Kassette der Vorrichtung angeordnet. Dadurch kann ein

Schutz vor Beschädigung gewährleistet werden und eine mechanisch stabile Anbringung ermöglicht werden. Ein einfacher Austausch der gesamten Kassette aus der Vorrichtung ist dadurch gewährleistet, und eine einfache Zugänglichkeit zur Diffusoranordnung ermöglicht. Die Diffusoranordnung ist dadurch des Weiteren quasi in einem größeren Gehäuse angeordnet, wodurch auch bei einem mehrfachen Herausnehmen und Wiedereinsetzen der Kassette in das System eine hohe Verschleißarmut gegeben ist.

Umfasst die Diffusoranordnung eine Mehrzahl von Diffusoren, so kann vorgesehen sein, dass zumindest ein Diffusor in der Kassette angeordnet ist.

Vorzugsweise weist zumindest ein Diffusor der Diffusoranordnung über seine Länge ein sich ausgehend von einem minimalen Innenmaß bis zu einem maximalen Innenmaß stetig vergrößerndes Innenmaß auf.

Bevorzugt ist eine Innenseite eines Diffusors der Diffusoranordnung in Längsrichtung des Diffusors zumindest bereichsweise gekrümmt ausgebildet. Es kann auch vorgesehen sein, dass eine Innenseite eines Diffusors der Diffusoranordnung in Längsrichtung des Diffusors zumindest bereichsweise gestuft ausgebildet ist.

Die Diffusoranordnung weist vorzugsweise ein Verhältnis K zwischen ihrem maximalen Innenmaß und ihrem minimalen Innenmaß auf, wobei das minimale und das maximale Innenmaß vorzugsweise an den Endöffnungen der Diffusoranordnung ausgebildet sind.

Ganz allgemein wird im vorliegenden Kontext unter einer Dämpfung bzw. Reduzierung, wie oben bereits erwähnt, weniger ein Dämpfen im klassischen Sinne verstanden, sondern insbesondere soll dadurch das Verändern der Übertragungscharakteristik der Vorrichtung für die Welle verstanden werden. Durch die Vorrichtung erfolgt somit eine Dämpfung im Sinne einer Verstimmung der Übertragungseigenschaften der Komponenten, was auch als Impedanztransformation bezeichnet wird. Es erfolgt durch die Diffusoranordnung eine Verschiebung der Resonanzen und es wird durch die Diffusoranordnung die Anregung einer stehenden Welle im relevanten Frequenzbereich in dem die Saugleitung, den Aspirationsschlauch, das Handstück, die Aspirationsleitung, die Diffusoranordnung und die Pumpe umfassenden Aspirationssystem verhindert. Je größer das Verhältnis K ist, umso effizienter kann dies erreicht werden.

Ein erfindungsgemäßes chirurgisches System, insbesondere ein ophthalmisches mikrochirurgisches System zur Phako-Chirurgie, umfasst eine im aktiven Betrieb nicht-kontinuierlich fördernde Pumpe, welche einem Aspirationszweig des Systems zugeordnet ist und vorzugsweise mit einer Aspirationsleitung des Aspirationszweigs verbunden ist. Darüber hinaus umfasst das chirurgische System ein chirurgisches Handstück, welches mit dem Aspirationszweig verbunden ist. Ferner weist das chirurgische System eine Vorrichtung nach Anspruch 1 zur Reduzierung von durch die im Betrieb der Pumpe erzeugten Druckschwankungen eines in dem Aspirationszweig des chirurgischen Systems strömenden Fluids auf, welche Vorrichtung eine Diffusoranordnung umfasst, die im Aspirationszweig in Strömungsrichtung des Fluids vor der Pumpe angeordnet ist. Insbesondere bei Kataraktoperationen, bei denen mittels Phako-Emulsifikation eine Linsenentfernung im Auge erfolgt, kann dadurch eine Operationsbeeinträchtigung aufgrund von Druckschwankungen des Fluids verhindert werden.

Vorteilhafte Ausführungen der erfindungsgemäßen Vorrichtung zum Dämpfen von Druckschwankungen sind als vorteilhafte Ausführungen des chirurgischen Systems anzusehen.

**Kurze Beschreibung der Zeichnungen**

Ausführungsbeispiele der Erfindung werden nachfolgend anhand schematischer Zeichnungen näher erläutert. Es zeigen:

Fig. 1          eine schematische Darstellung von Teilelementen eines erfindungsgemäßen chirurgischen Systems;

Fig. 2          eine schematische Darstellung eines Teilausschnitts des chirurgischen Systems gemäß Fig. 1;

Fig. 3a bis 3i   Ausführungen einer Diffusoranordnung einer erfindungsgemäßen Vorrichtung;

Fig. 4a bis 4f   Längsschnittdarstellungen verschiedener Formgebungen einer Diffusoranordnung;

Fig. 5a bis 5k   Querschnittdarstellungen verschiedener Formgebungen einer Diffusoranordnung;

Fig. 6          ein aus dem Stand der Technik bekanntes Modell eines chirurgischen Systems;

Fig. 7          ein strömungstechnisches Ersatzschaltbild für eine Komponente des Modells gemäß Fig. 6;

Fig. 8          ein Ausführungsbeispiel eines Modells eines erfindungsgemäßen chirurgischen Systems;

Fig. 9          ein strömungstechnisches Ersatzschaltbild für eine Komponente des Modells gemäß Fig. 8;

Fig. 10         Diagramme, welche die Drehfrequenzen der Pumpe, die Amplituden der Druckwelle und den Augeninnendruck in Abhängigkeit von der Zeit bei einem Verhältnis K=1 zeigen;

Fig. 11  Diagramme, welche die Drehfrequenzen der Pumpe, die Amplituden der Druckwelle und den Augeninnendruck in Abhängigkeit von der Zeit bei einem Verhältnis K=15 zeigen;

Fig. 12  Diagramme, welche die Drehfrequenzen der Pumpe, die Amplituden der Druckwelle und den Augeninnendruck in Abhängigkeit von der Zeit bei einem Verhältnis K=20 zeigen;

Fig. 13  Diagramme, welche die Drehfrequenzen der Pumpe, die Amplituden der Druckwelle und den Augeninnendruck in Abhängigkeit von der Zeit bei einem Verhältnis K=30 zeigen;

Fig. 14  ein Diagramm, in welchem ein prozentuales Amplitudenverhältnis der Druckwelle in Abhängigkeit des Verhältnisses K dargestellt ist;

Fig. 15  ein Diagramm, in welchem die Wellenausbreitungsgeschwindigkeit in Abhängigkeit von der Länge der Diffusoranordnung für verschiedene Werte des Verhältnisses K dargestellt ist;

Fig. 16  eine erste Tabelle, in welcher Parameterwerte für eine Simulation des in Fig. 6 gezeigten Modells gezeigt sind;

Fig. 17  eine zweite Tabelle, in welcher Werte von Parametern und Variablen für eine Simulation eines in Fig. 8 gezeigten Modells für ein erstes Ausführungsbeispiel eines erfindungemäßen chirurgischen Systems gezeigt sind;

Fig. 18  eine dritte Tabelle, in welcher Werte von Parametern und Variablen für eine Simulation eines in Fig. 8 gezeigten Modells für ein zweites Ausführungsbeispiel eines erfindungemäßen chirurgischen Systems gezeigt sind;

Fig. 19  eine vierte Tabelle, in welcher Werte von Parametern und Variablen für eine Simulation eines in Fig. 8 gezeigten Modells für ein drittes Ausführungsbeispiel eines erfindungemäßen chirurgischen Systems gezeigt sind.

## Bevorzugte Ausführung der Erfindung

In den Figuren werden gleiche oder funktionsgleiche Elemente mit den gleichen Bezugszeichen versehen.

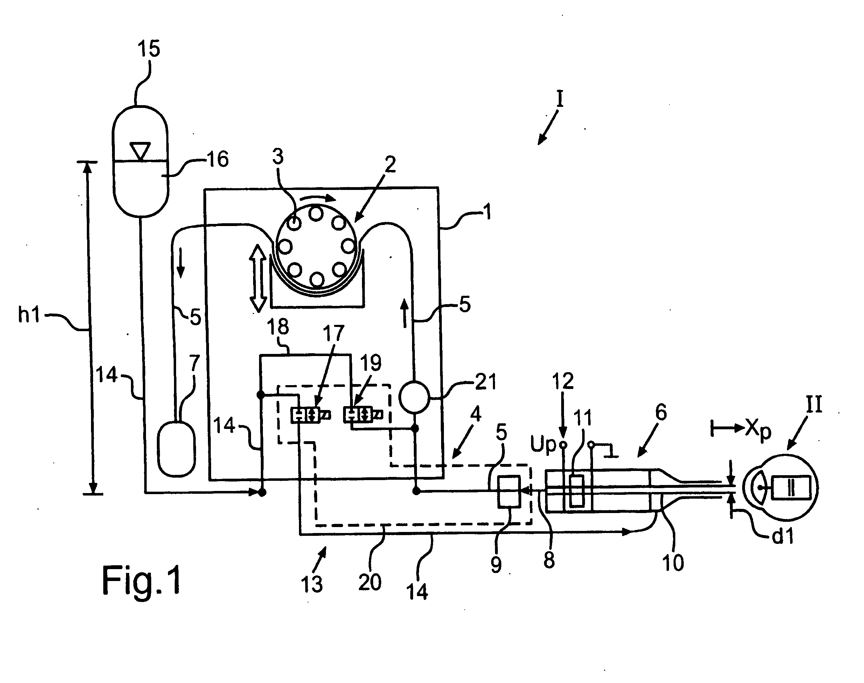

In einer schematischen Darstellung ist in Fig. 1 ein erfindungsgemäßes ophthalmisches mikrochirurgisches System I zur Phako-Chirurgie gezeigt. In der Darstellung gemäß Fig. 1 sind lediglich die für das Verständnis der Erfindung wesentlichen Komponenten des Systems I gezeigt.

Das chirurgische System I umfasst eine Geräteeinheit 1, welche beispielsweise als Rollwagen oder dergleichen ausgebildet sein kann und neben elektrischen Steuereinheiten, elektronischen Treibereinheiten für mikrochirurgische Instrumente, einer Benutzerschnittstelle und einer Anzeigeeinheit auch eine Pumpe 2 aufweist. Diese Pumpe 2 ist im Ausführungsbeispiel als Peristaltikpumpe ausgebildet und ist somit eine im aktiven Betrieb nicht-kontinuierlich fördernde Pumpe. Die Pumpe 2 umfasst mehrere Rollenräder 3, welche beabstandet zueinander in Umlaufrichtung (Pfeil) angeordnet sind und jeweils eine abgeschlossene Volumenkammer aus dem Saugbereich entziehen.

Die Pumpe 2 ist in einem Aspirationszweig 4 des chirurgischen Systems I angeordnet und fördert die in einer Aspirationsleitung 5 des Aspirationszweigs 4 strömenden Flüssigkeit von einem chirurgischen Handstück 6, welches im Ausführungsbeispiel das Phako-Handstück ist, bis zu einem Sammelbehälter 7. Der Aspirationszweig 4 umfasst in der gezeigten Ausführung auch einen elastischen Aspirationsschlauch 8 (ASP-Schlauch), welcher sich zwischen dem Handstück 6 und einer im Aspirationszweig 4 angeordneten Diffusoranordnung 9 erstreckt. Die Diffusoranordnung 9 ist vorzugsweise möglichst nahe bei der Pumpe 2 angeordnet.

Die Pumpe 2 ist aufgrund ihrer Bauart so konzipiert, dass die Rollenräder 3 kontaktfrei mit dem in der Aspirationsleitung 5 strömenden Fluid ausgebildet sind und somit keine direkte Verbindung zwischen den Rollenrädern 3 und dem Fluid gegeben ist.

Das Handstück 6 umfasst eine Spitze bzw. Hohlnadel 10, welche über einen Ultraschallgeber 11 zur Oszillation angeregt wird. Der Ultraschallgeber 11 kann beispielsweise ein Piezo sein, welcher über eine elektrische Spannung zur Oszillation angeregt wird. Die Spannung wird durch eine symbolisch gezeigte Spannungsquelle 12 erzeugt. Die Hohlnadel 10 ist an ihrer dem Auge II zugewandten vorderen Seite mit einer Öffnung versehen, welche einen Durchmesser $d_1$ aufweist. Der Durchmesser $d_1$ ist kleiner als der Durchmesser der restlichen Hohlnadel 10, wodurch die Hohlnadel 10 an dieser vorderen Öffnung verjüngt ausgebildet ist.

Darüber hinaus umfasst das chirurgische System I einen Irrigationszweig 13 mit einer Irrigationsleitung 14, welche sich zwischen einem Behälter 15 mit einer Spülflüssigkeit 16 und dem Handstück 6 erstreckt. Die Spülflüssigkeit 16 kann beispielsweise eine Salzlösung (BSS-Lösung) sein. Wie aus der Darstellung in Fig. 1 zu erkennen ist, befindet sich der Behälter 15 im Vergleich zum Handstück 6 auf einem Höhenniveau h1, wodurch abhängig von dieser Höhe h1 der Druck der Spülflüssigkeit 16 in der Irrigationsleitung 14 verändert werden kann. In der Irrigationsleitung 14 ist des Weiteren ein Ventil 17 angeordnet. Die Irrigationsleitung 14 ist mit der Aspirationsleitung 5 durch eine Schlauchverbindung 18 verbunden, wobei in dieser Schlauchverbindung 18 ein weiteres Ventil 19 angeordnet ist.

Die Irrigationsleitung 14 ist vorzugsweise zumindest teilweise als elastischer Schlauch (IRR-Schlauch) ausgebildet, welcher sich zwischen dem Handstück 6 und einer Fluidik-Kassette 20 erstreckt. Die Kassette 20 kann die Diffusoranordnung 9 zumindest teilweise umfassen. Ebenso kann vorgesehen sein, dass die Kassette 20 die Ventile 17 und 19 umfasst. Die Irrigationsleitung 14 kann auch zwischen dem Behälter 15 und der Kassette 20 als elastischer Schlauch (BSS-Schlauch) ausgebildet sein. Der sich in der Kassette 20 befindende Teil der Aspirationsleitung 5 wird nachfolgend auch als ASP-Leitung bezeichnet.

Im Aspirationszweig 4 ist des Weiteren ein Druckmesser 21 angeordnet, welcher im Ausführungsbeispiel in Strömungsrichtung des Fluids in der Aspirationsleitung 5 der Verbindungsleitung 18 zur Irrigationsleitung 14 nachgeschaltet ist.

Die über die Irrigationsleitung 14 zugeführte Spülflüssigkeit 16 wird während dem chirurgischen Eingriff am Auge II über die Hohlnadel 10 mit den zertrümmerten Linsenresten über den Aspirationszweig 4 durch die Pumpe 2 abgesaugt.

Das chirurgische System I umfasst des Weiteren eine Vorrichtung zur Reduzierung bzw. Dämpfung von Druckschwankungen des in dem Aspirationszweig 4 strömenden Fluids, wobei die Druckschwankungen durch die nichtkontinuierliche Förderung der Pumpe 2 erzeugt werden. Diese Vorrichtung zur Dämpfung der Druckschwankungen umfasst die Diffusoranordnung 9, die im Aspirationszweig 4 in Strömungsrichtung (Pfeil) des Fluids vor der Pumpe 2 angeordnet ist.

Die Diffusoranordnung 9 ist in Fig. 1 lediglich mittels eines symbolischen Blockellements gezeigt und relativ nahe am Handstück 6 angeordnet. In der gezeigten Ausführung ist die Diffusoranordnung 9 beabstandet zum Handstück 6 und auch beabstandet zur Pumpe 2 angeordnet. Es kann auch vorgesehen sein, dass die Diffusoranordnung 9 zumindest bereichsweise in dem Handstück 6 angeordnet ist oder unmittelbar am in den Aspirationsschlauch 8 mündenden hinteren Ende des Handstücks 6 angeordnet ist.

Durch die Vorrichtung zur Dämpfung dieser Druckschwankungen mit der Diffusoranordnung 9 kann das Frequenzspektrum bzw. das Wellenlängen-/Amplitudenspektrum dieser Druckpulsationen der strömenden Flüssigkeit im Aspirationszweig 4 gedämpft werden. Insbesondere kann durch diese Anordnung auch die Dämpfungswirkung des elastischen Aspirationsschlauchs 8 besser zur Wirkung kommen.

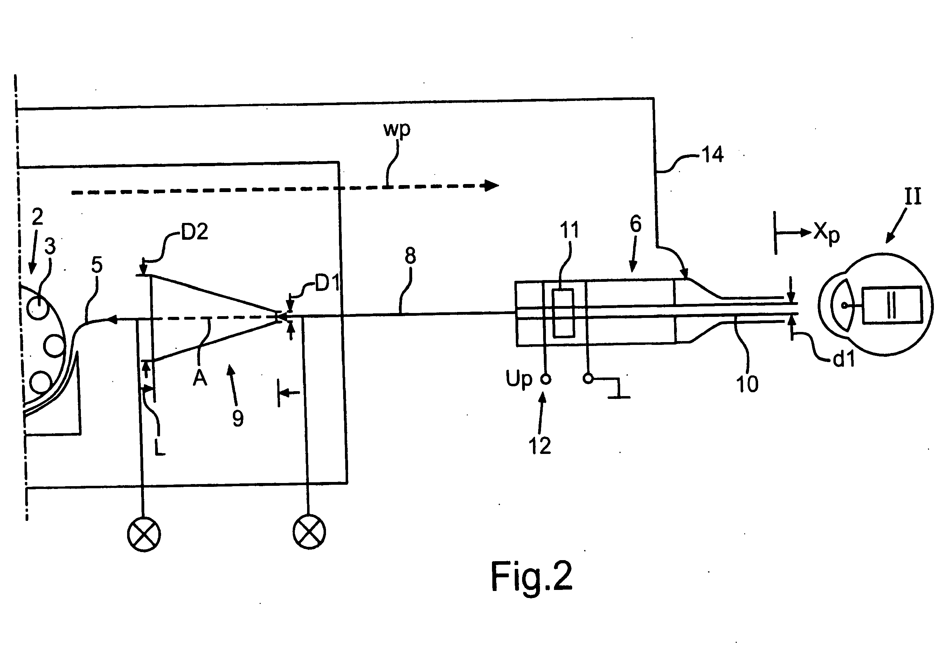

In Fig. 2 ist eine schematische Darstellung eines Teilausschnitts des chirurgischen Systems I gemäß Fig. 1 gezeigt. Die Diffusoranordnung 9 umfasst bei dieser beispielhaften Ausführung lediglich einen Diffusor, welcher trichterförmig ausgebildet ist und sich in Strömungsrichtung des Fluids im Aspirationszweig 4 aufweitet. An der dem Handstück 6 zugewandten Seite des Diffusors weist dieser ein minimales Innenmaß D1 auf. Insbesondere kann vorgesehen sein, dass der Diffusor im Querschnitt im Wesentlichen rund ist, so dass das Innenmaß der Innendurchmesser ist. Dieses minimale Innenmaß D1 ist gleich oder größer dem minimalen Innenmaß, insbesondere dem Innendurchmesser, d1 der Hohlnadel 10. Das minimale Innenmaß D1 der Diffusoranordnung 9 beträgt insbesondere zwischen 1 mm und 2 mm.

An der der Pumpe 2 zugewandten Endseite der Diffusoranordnung 9 weist der Diffusor ein maximales Innenmaß D2, insbesondere einen Innendurchmesser, auf, welcher im Ausführungsbeispiel zugleich das maximale Innenmaß des Diffusors über seine Länge L darstellt.

Die Innenmaße D1 und D2 sind in der gezeigten Ausführung an den Endöffnungen der Diffusoranordnung 9 bzw. des einzigen Diffusors definiert.

Das maximale Innenmaß D2 beträgt insbesondere zwischen 1,5 mm und 10 mm.

Darüber hinaus weist die Diffusoranordnung 9 und in der gezeigten Ausführung somit auch der einzige Diffusor eine Länge L auf, welche abhängig von spezifischen Parametern des Fluids und/oder von dem Aspirationszweig 4 zugeordneten Komponenten, beispielsweise dem Aspirationsschlauch 8, und/oder der Pumpe 2 und/oder der Diffusoranordnung 9 selbst vorgegeben sein kann. Insbesondere die Formgebung, die Abmessungen, das Material und die Anordnung der Komponenten zueinander können diesbezüglich die Länge L beeinflussen. Um eine optimale Dämpfung der Druckschwankungen erreichen zu können ist jedoch insbesondere das Verhältnis K zwischen dem maximalen und dem minimalen Innenmaß D2 bzw. D1 optimal zu dimensionieren.

Diese Bestimmung der Größe K und der Formgebung der Diffusoranordnung 9 kann bevorzugt durch eine Simulationsrechnung erhalten werden.

In der in Fig. 2 gezeigten Ausführung des einzigen Diffusors der Diffusoranordnung 9 ist dieser bezüglich der Rotationsachse A symmetrisch ausgebildet.

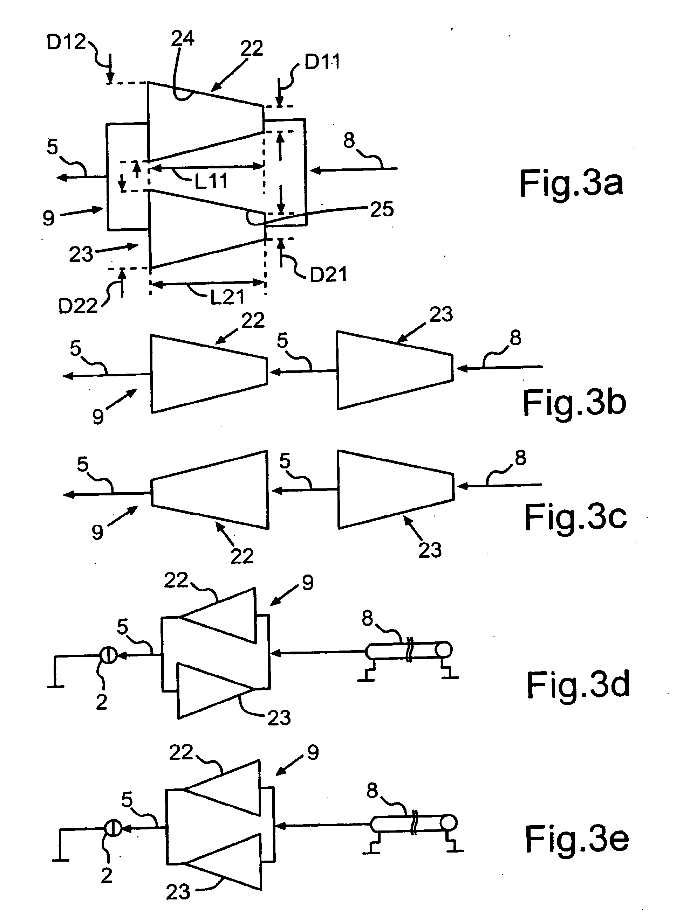

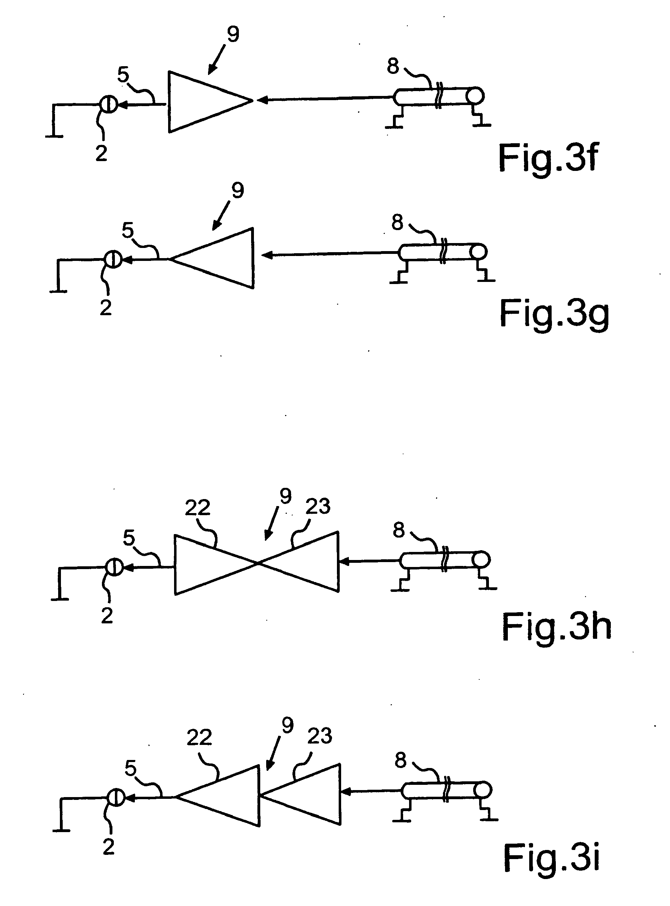

In den Fig. 3a bis 3i sind weitere beispielhafte Ausführungen einer Diffusoranordnung 9 gezeigt, wobei selbstverständlich auch darüber hinausgehende und nicht gezeigte Ausgestaltungen einer Diffusoranordnung 9 durch die Erfindung umfasst sind.

In Fig. 3a ist ein weiteres Ausführungsbeispiel einer Diffusoranordnung 9 in schematischer Weise dargestellt. Bei dieser Ausführung umfasst die Diffusoranordnung 9 zumindest zwei Diffusoren 22 und 23, welche parallel geschaltet sind. In der gezeigten Ausführung sind beide Diffusoren 22 und 23 gleich ausgebildet und weisen somit gleiche Formgebung und gleiche Ausmaße auf und sind aus dem gleichen Material ausgebildet. Die beiden Diffusoren 22 und 23 sind so orientiert, dass die jeweils dem Handstück 6 zugewandten Enden den minimalen Innendurchmesser aufweisen. Der erste Diffusor 22 weist ein minimales Innenmaß D11 und der zweite Diffusor 23 weist ein minimalen Innenmaß D21 auf. Beide Diffusoren 22 und 23 sind so orientiert und ausgebildet, dass die jeweils der Pumpe 2 zugewandten Enden das maximale Innenmaß aufweisen. So weist der erste Diffusor 22 ein maximales Innenmaß D12 und der zweite Diffusor 23 ein maximales Innenmaß D22 auf. Darüber hinaus weist der erste Diffusor 22 eine Länge L11 auf, welche der Länge L21 des zweiten Diffusors 23 entspricht. Beide Diffusoren 22 und 23 sind so ausgebildet, dass sie ausgehend von ihren dem Handstück 6 zugewandten Enden mit dem minimalen Innenmaß D11 und D21 bis hin zu den anderen Enden mit den maximalen Innenmaßen D12 und D22 sich stetig aufweitend ausgebildet sind. Darüber hinaus sind die Innenseiten 24 und 25 über die gesamte Länge zwischen den beiden Enden der Diffusoren 22 und 23 geradlinig und somit krümmungsfrei ausgebildet. Dadurch ergibt sich eine Kegelstumpfform für die beiden Diffusoren 22 und 23.

Es kann auch vorgesehen sein, dass die Diffusoren 22 und 23 in ihren Formgebungen und/oder in den Ausmaßen und/oder in den Materialien, aus denen sie gefertigt sind, unterschiedlich sind. Ebenso kann auch vorgesehen sein, dass beispielsweise der erste Diffusor 22 in einer um 180 Grad gedrehten Anordnung in der Parallelschaltung angeordnet ist. Dies ist beispielhaft und symbolisch in Fig. 3d gezeigt. Bei dieser Ausführung wäre dann das Ende mit dem maximalen Innenmaß D12 dem Handstück 6 zugewandt.

In Fig. 3b ist eine weitere Ausführung einer Diffusoranordnung 9 gezeigt, bei der zumindest zwei Diffusoren 22 und 23 in Serie zueinander geschaltet sind. Die beiden Diffusoren 22 und 23 sind in der beispielhaften Darstellung analog zu den Diffusoren 23 und 23 in Fig. 3a ausgebildet. In Fig. 3b sind die Diffusoren 22 und 23 beabstandet zueinander in dem Aspirationszweig 4 angeordnet.

Es kann auch vorgesehen sein, dass die Diffusoren 22 und 23 unmittelbar aneinander angrenzend angeordnet sind. Auch hier kann wieder eine Unterschiedlichkeit der Diffusoren 22 und 23 in Formgebung und/oder Ausmaßen und dergleichen vorgesehen sein.

In Fig. 3c ist eine weitere Ausführung einer Serienschaltung von Diffusoren 22 und 23 einer Diffusoranordnung 9 gezeigt, bei der die Diffusoren 22 und 23 mit den Enden, welche die maximalen Innenmaße aufweisen, einander zugewandt sind. Die gesamte Diffusoranordnung 9 weist somit eine Konstruktion auf, welche zunächst ein sehr kleines Innenmaß aufweist, welches sich dann bis zu einem maximalen Innenmaß aufweitet und ausgehend von diesem maximalen Innenmaß sich dann wieder bis auf ein kleines, insbesondere minimales, Innenmaß verjüngt. Auch bei dieser Ausgestaltung in Fig. 3c kann vorgesehen sein, dass die beiden Diffusoren 22 und 23 nicht beabstandet zueinander angeordnet sind, sondern unmittelbar aneinander angrenzen.

Die Innenwände 24 und 25 der Diffusoren 22 und 23 können zumindest teilweise elastisch für Krafteinwirkungen sein, wie sie durch Druckpulsationen des hindurchströmenden Fluids erzeugt werden können.

Ebenso kann vorgesehen sein, dass diese Innenseiten 24 bzw. 25 vollständig starr und somit unverformbar gegenüber derartigen Krafteinwirkungen sind.

In den Fig. 3e bis 3i sind weitere beispielhafte Ausgestaltungen einer Diffusoranordnung 9 gezeigt, welche durch entsprechende Symbole dargestellt sind. Dabei sind weitere Ausführungen von Parallelschaltungen und Serienschaltungen von zumindest zwei Diffusoren einer Diffusoranordnung 9 gezeigt. Prinzipiell ist die Anzahl der Diffusoren nicht auf einen oder zwei beschränkt, sondern es kann auch eine größere Anzahl vorgesehen sein.

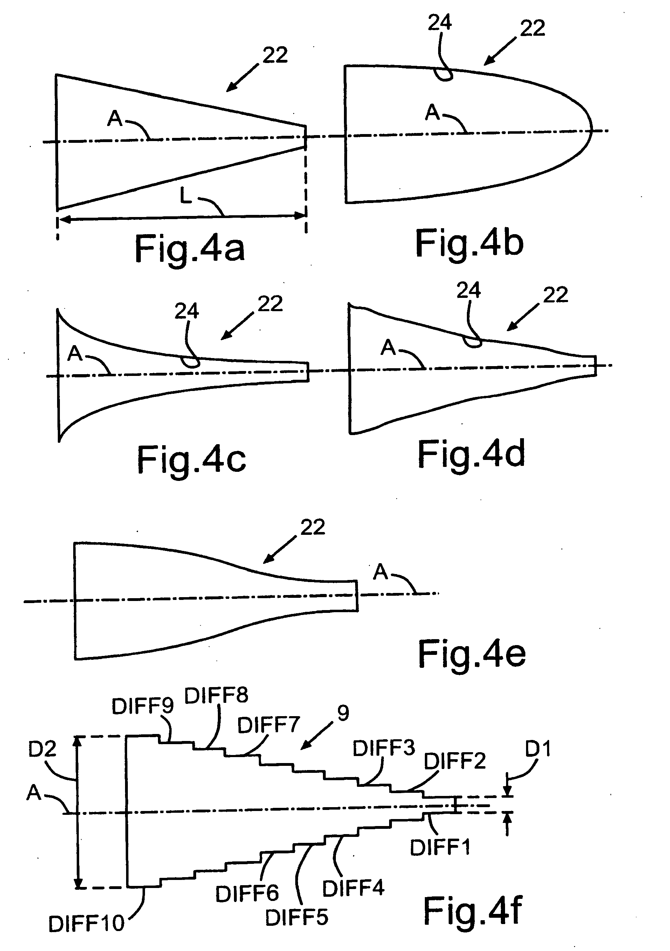

In den Fig. 4a bis 4f sind Ausführungen von Längsschnittdarstellungen einer Diffusoranordnung 9 gezeigt.

In Fig. 4a ist eine Schnittdarstellung durch eine Diffusoranordnung 9 gezeigt, welche einen Diffusor 22 mit einer streng kegelstumpfförmigen Formgebung aufweist. Dies bedeutet, dass die Innenseite 24 über die Länge L in der gezeigten Schnittdarstellung quasi eine gerade Linie darstellt, welche schräg zwischen den Enden des Diffusors 22 verläuft.

In Fig. 4b ist eine weitere beispielhafte Formgebung eines Diffusors 22 in einer Längsschnittdarstellung gezeigt. Bei dieser Ausführung weist die Innenseite bzw. die Mantelfläche der Innenseiten 24 einen in Richtung der Achse A betrachtet gekrümmten Verlauf auf. Die Krümmung weist dabei einen quasi nach außen hin gewölbten Verlauf auf.

In Fig. 4c ist eine weitere Schnittdarstellung eines Ausführungsbeispiels eines Diffusors 22 gezeigt, welcher ebenfalls eine gekrümmt verlaufende Innenseite 24 aufweist. Ebenso wie in der Ausführung gemäß Fig. 4b ist auch hier die Krümmung über die gesamte Länge L ausgebildet, wobei sie jedoch bei der Ausführung in Fig. 4c nicht nach außen gewölbt sondern nach innen gewölbt ausgeführt ist.

Eine weitere Ausführung einer Formgebung der Innenseite 24 eines Diffusors 22 ist in der Längsschnittdarstellung gemäß Fig. 4d gezeigt. Bei dieser Ausführung weist die Innenseite 24 einen mehrfach gewellten Verlauf über die Länge L auf, wodurch quasi eine Freiformkontur gestaltet ist. In der Schnittdarstellung ist die Innenseite 24 somit wellenförmig ausgestaltet.

In Fig. 4e ist eine weitere Form gezeigt, bei der eine zur Achse A symmetrische Ausgestaltung ausgebildet ist und etwa mittig eine stärkere Verjüngung realisiert ist. In Fig. 4f ist eine gestufte Formgebung dargestellt, welche zehn verschieden Teilsegmente DIFF1 bis DIFF10 umfasst. Eine derartige Ausgestaltung ist auch im Hinblick auf eine Modellrechnung mit einer Simulation als Hilfskonstruktion geeignet. Die Anzahl der Teilsegmente ist lediglich beispielhaft und kann in vielfältiger Weise anderweitig ausgebildet sein. Insbesondere bei einem sehr komplexen Konturenverlauf kann auch eine sehr große Anzahl von Teilsegmenten vorgesehen sein, welche dann in Richtung der Achse A und somit in Längsrichtung der Diffusoranordnung 9 minimale Teillängen aufweisen, wobei dann durch eine relativ geringe Innemaßveränderung zwischen zwei aufeinander folgenden Teilsegmenten ein stufenfreier Konturenverlauf erreicht und integral berechnet werden kann. Die Summe der Teillängen ergibt dann die gesamte Länge L.

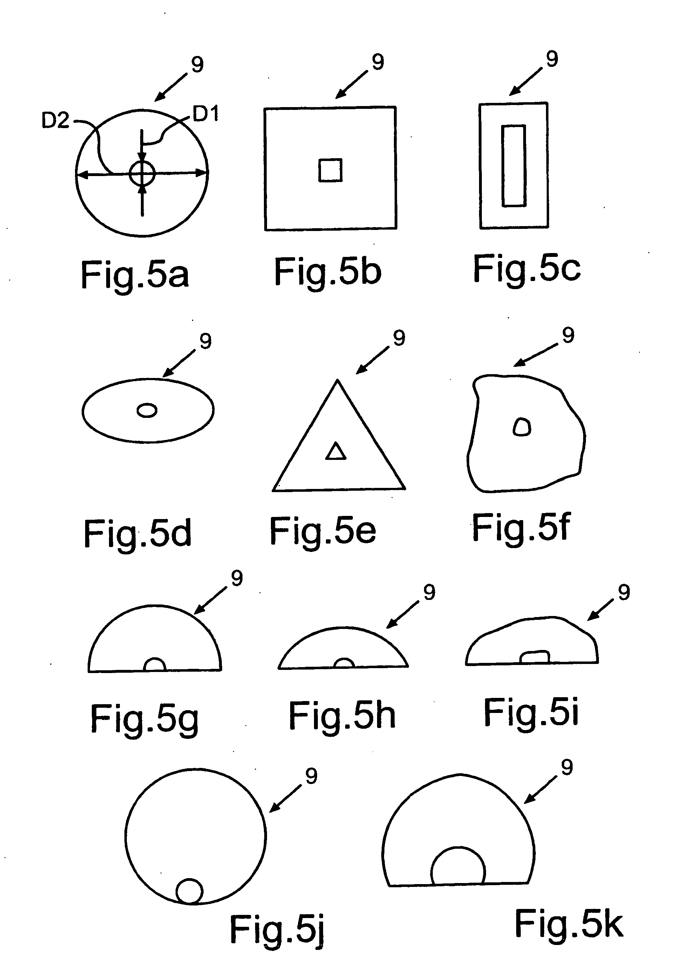

In Fig. 5a bis 5k sind Querschnittdarstellungen von verschiedenen Ausführungen einer Diffusoranordnung 9 gezeigt.

Die Fig. 5a bis 5k zeigen schematische Ansichten, die dabei so gewählt sind, dass in Längsachsenrichtung A von dem Ende mit dem größeren Innenmaß in Richtung zu dem anderen Ende mit dem kleineren Innenmaß gesehen wird. Fig. 5a zeigt dabei eine vollkommen runde Formgebung der beiden Endbereiche mit den Innendurchmessern D2 und D1.

In Fig. 5b ist eine Ausführung mit eckigen, insbesondere quadratischen, Endformgebungen gezeigt.

In Fig. 5c ist wiederum eine Formgebung gezeigt, bei der die Endbereiche viereckig geformt sind, jedoch hier in einer Rechteckform.

Fig. 5d zeigt eine eckenfreie Ausgestaltung der Enden der Diffusoranordnung 9, welche jedoch abgeflacht, insbesondere oval, ausgebildet sind.

In Fig. 5e ist eine Dreieckformgebung der Enden gezeigt.

Fig. 5f zeigt eine Ausführung, bei der die gegenüberliegenden Enden der Diffusoranordnung 9 unterschiedliche Formgebungen aufweisen, welche jeweils eine Freiformkontur zeigen.

In Fig. 5g ist eine im Hinblick auf eine horizontale Achse nicht symmetrische Ausgestaltung gezeigt, welche jeweils Halbkreise für die beiden Enden der Diffusoranordnung 9 zeigen. Eine ähnliche Formgebung zeigt Fig. 5h, wobei Fig. 5i wiederum Freiformkonturen der beiden Enden zeigt.

In den Fig. 5j und 5k sind weitere asymmetrische Ausführungen gezeigt, wobei in Fig. 5j die Innenmaße D1 und D2 Innendurchmesser der runden Querschnitte sind, und in Fig. 5k die Konturen der Endöffnungen der Diffusoranordnung 9 teilweise gekrümmt sind und und auch Ecken aufweisen.

Allgemein kann durch die Erfindung erreicht werden, dass die Wellengeschwindigkeit und insbesondere die Amplituden von Druckschwankungen bzw. Druckwellen des strömenden Fluids im Aspirationszweig im Bereich der Diffusoranordnung der Vorrichtung zur Dämpfung dieser Druckschwankungen deutlich reduziert werden kann bzw. können.

Die als Peristaltikpumpe ausgebildete Pumpe 2 kann beispielsweise acht Rollenräder 3 aufweisen. Beispielsweise kann sie eine Nenndrehzahl von 600 U/min und eine Fördermenge von 60 ml/min aufweisen. Dabei erstreckt sich der Nennbereich der Pulsationsfrequenz beispielsweise von 0 bis etwa 80 Hz.

Bevorzugt kann als Material für eine Diffusoranordnung 9 bzw. einen Diffusor 22 oder 23 Polycarbonat verwendet werden. Durch diese Materialauswahl kann eine kostengünstige und einfach zu fertigende Ausgestaltung ermöglicht werden. Insbesondere kann die Diffusoranordnung 9 als Spritzgussteil gefertigt sein. Durch diese Materialwahl und diese Fertigungsweise können auch vielfältig gestaltete und komplexeste Formgebungen einer Diffusoranordnung 9 ermöglicht werden. Darüber hinaus kann durch diese Materialien eine sehr leichte Diffusoranordnung 9 ermöglicht werden. Neben einem Polycarbonat kann auch ein Silikonmaterial, welches sowohl als hartes als auch als weiches Silikonmaterial ausgebildet sein kann, verwendet werden.

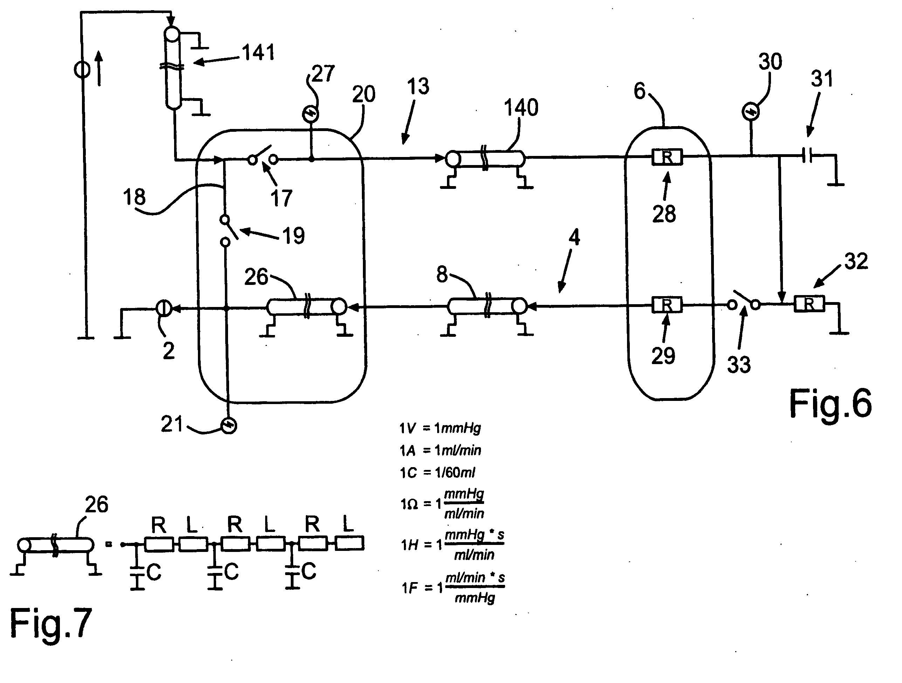

Fig. 6 zeigt in einem Modell für eine Simulation ein Standard Operationssystem mit einem Operationshandstück. Das Fluidiksystem für dieses Phako-Emulsifikationssystem gliedert sich in zwei Funktionsgruppen. Das Irrigationssystem führt während der Kataraktoperation die Bewässerung des Auges mit BSS Lösung durch. Simultan wird über ein Pumpenaspirationssystem das durch den Ultraschallprozess emulsifizierte Kataraktmaterial abgesaugt. Das Fluidik Modul ist mit diesen beiden Funktionen über ein flexibles Schlauchsystem mit dem Phaco Handstück verbunden.

Ausgangspunkt für das Irrigationssystem ist die Austauschspülflüssigkeit BSS. Die BSS Lösung wirkt durch die in der Höhe positionierbare Flasche als hydrostatische konstante Druckquelle für das Irrigationssystem. Die BSS Flasche ist mit der Fluidik des Gerätes über einen flexiblen elastischen BSS Schlauch 141 verbunden.

Der hydrostatische Druck kann nach dem physikalischen Gesetz bestimmt werden:

$$p = \rho \cdot g \cdot h$$

Die Dichte ρ, die Erdbeschleunigung g sowie die Flaschenhöhe h, im Ausführungsbeispiel gemäß Fig. 1 ist dies h1, bestimmen jeweils als lineare Einflussgröße den hydrostatischen Druck p des Irrigationssystems des chirurgischen Systems I. Bei der Dichte ρ der BSS Lösung wird bei allen Betrachtungen von ρ=1000 kg/m$^3$ ausgegangen. Bei allen weiteren Betrachtungen wird ein hydrostatischer Druck durch die Höhe der BSS Flasche von 50 mmHg angenommen.

Im Fluidiksystem des chirurgischen Systems I wird der Saugfluss Q über die Pumpe 2 realisiert. Während des Absaugens wird der Saugdruck p im Aspirationszweig 4 gemessen (Sensor in der Kassette 20) und für die Überwachung und Steuerung der Fluidik- und Ultraschallsysteme eingesetzt. Die Pumpe 2 fördert durch ihr Bauartprinzip keinen kontinuierlichen Flow Q(t), da die Öffnungs- bzw. Schließventile bzw. die Rollenräder 3 jeweils ein abgeschlossenes Volumeninkrement aus dem Aspirationsbereich entziehen. Aus diesem Grund induziert die Pumpe 2 im Aspirationszweig des Operationsgerätes durch ihre Flowpulse Q(t) Druckpulsationen p(t).

Diese Druckpulsationen p(t) breiten sich als Longitudinalwellen entlang des Aspirationszweigs 4 in Richtung des zu operierenden Auges II fort. Sie werden im Bereich der Aspirationsleitung des Handstückes 6 zu einem Teil reflektiert. Ein gewisser Anteil dieser Pulsationswellen gelangt jedoch über die Aspirationsleitung des Handstückes 6 in die durch den Operationsschnitt geöffnete Augenkammer. Der intraokulare Druck wird dadurch ebenfalls zu Druckschwingungen in der Augenkammer angeregt. Dies ist eine für den Operationsverlauf sehr ungünstige Eigenschaft. Der Operateur bemerkt die Druckschwingungen als pulsierendes Auge. Der sehr feinfühlige Eingriff wird dadurch sehr gestört.

Durch die Erfindung wird eine Lösung gefunden, welche die Pulsationsanregung im Auge durch die Druckwellen im Aspirationszweig 4 zumindest stark vermindern kann.

Nach dem Stand der Technik könnte dies durch die Anordnung einer Kapazität in der Nähe der die Pulsationen anregenden Pumpe 2 oder durch die Erhöhung der Elastizität der Aspirationsleitung geschehen. Dies muss jedoch aus verfahrenstechnischen Gründen auf jeden Fall vermieden werden, da im Falle einer Okklusion der Aspirationsnadel im Durchbruch der Okklusion die unter sehr hoher Vakuumvorspannung stehende elastische Aspirationsleitung sowie der elastische Aspirationsschlauch gefährlich viel Flüssigkeit kurzzeitig aus der Augenkammer saugen können. Diese unter dem Begriff "Surge" bekannte Druckwelle gefährdet den Erfolg der Augenoperation und muss vermieden werden. Weiterhin können durch diese impulsartigen Sogkräfte an der Spitze der Hohlnadel 10 des Handstückes 6 Schnittverletzungen des hinteren Augenkapselsackes auftreten. Aus diesem Grund muss der Aspirationszweig 4 vom Handstück 6 bis zur Pumpe 2 mit einer geringst möglichen Elastizität ausgestattet werden.

Die Art der Pulsationsdämpfung durch eine Erhöhung der Elastizität der Leitung und Schläuche im Aspirationszweig bzw. der Anordnung einer zusätzlichen Kapazität ist deshalb auszuschließen.

Eine weitere Möglichkeit zur Druckwellendämpfung nach dem Stand der Technik wäre die Erhöhung des hydraulischen Widerstands durch die Verminderung der Aspirationsleitungsquerschnitte. Da aus dem Auge jedoch eine Emulsion abtransportiert werden soll, sollte der komplette InnenQuerschnitt der Leitungen und Schläuche im Aspirationszweig 4 deutlich größer ausgeführt werden, wie der engste Querschnitt in der Hohlnadel 10, damit das Aspirationsleitungssystem nicht verstopfen kann und der Druckverlust durch die Flowströmung nicht zu hoch wird. Dies Möglichkeit ist deshalb ebenfalls auszuschließen.

Nachfolgend soll kurz erläutert werden, wie anhand eines hydraulischen Simulationsmodells die auftretenden longitudinalen Druckwellen im Aspirationszweig 4 sowie die daraus angeregten Druckschwingungen im Auge II berechnet und dargestellt werden können.

Der hydraulische Widerstand $R_h$ einer Leitung kann bei laminarer Rohrströmung nach dem Hagen-Poiseuille'schen Gesetz bestimmt werden zu:

$$R_h = \frac{8 \cdot \eta \cdot l}{\pi \cdot r^4}$$

Der Innenradius r der Leitung geht hierbei mit dem Kehrwert der vierten Potenz ein. Die Länge *l* der Leitung sowie die dynamische Viskosität η der Flüssigkeit gehen dabei jeweils linear ein. Die Einheit des hydraulischen Widerstandes kann z.B. in [mmHg/ml*min] ausgedrückt werden.

Die hydraulische Leitung besitzt eine Induktivität $L_h$. Die hydraulische Induktivität charakterisiert die dynamische Druckänderung auf Grund der Massenträgheit des zu beschleunigenden Flüssigkeitsvolumens. Sie kann nach folgender Formel bestimmt werden:

$$L_h = \frac{\rho \cdot l}{A}$$

Die Dichte ρ der Flüssigkeit bestimmt mit der Leitungslänge 1 sowie mit dem inneren Leitungsquerschnitt A die hydraulische Induktivität $L_h$, deren Einheit in [mmHg*s /(ml/min)] ausgedrückt werden kann.

Die elastischen Eigenschaften der Schlauchleitung werden in erster Näherung durch die Materialeigenschaften des Wandungsmaterials bestimmt. Bei einem weichen Silikonschlauch geht man von einem Elastizitätsmodul von $E_{weich}$ = 1 MPa aus, ein harter PVC bzw. Silikonschlauch weist einen E-Modul von $E_{hart}$ = 3 MPa auf. Die hydraulische Kapazität $C_h$ kann somit über die elastischen Eigenschaften des Schlauch- bzw. Wandungsmaterials der Leitungen $E_{Wandung}$ nach folgender Gleichung bestimmt werden:

$$C_h = \frac{V_0 \cdot 2 \cdot r}{E_{Wandung} \cdot h}$$

$V_0$ bezeichnet dabei das durch die Leitung umschlossene Flüssigkeitsvolumen. Die Wandstärke h der Leitungswandung sowie der Innenradius $r$ der Leitung und der E-Modul $E_{Wandung}$ der elastischen Wandung bestimmen die kapazitiven Eigenschaften der elastischen Leitung mit der möglichen Einheit [ml/min*s / mmHg].

Ausgehend von der hydraulischen Kapazität $C_h$ und der Induktivität $L_h$ einer elastischen Leitung kann die Ausbreitungsgeschwindigkeit $c$ der Druckwellen nach folgender Formel bestimmt werden zu:

$$c = \frac{l}{\sqrt{L_h \cdot C_h}} = \sqrt{\frac{E_{Wandung} \cdot h}{2 \cdot r \cdot \rho}}$$

Die Einheit der Wellenausbreitungsgeschwindigkeit bzw. Phasengeschwindigkeit c ist [m/s]. Wie zu erkennen ist, kürzen sich Längen bei der Formel für die Wellenausbreitungsgeschwindigkeit c gemäß dem hinteren Ausdruck in der Formel heraus.

Für einen Aspirationsschlauch mit einem Innenradius $r$ = 0.9 mm sowie mit einem Elastizitätsmodul von $E_{Wandung}$ = 3 MPa für einen harten PVC-Schlauch und einer Wandstärke $h$ = 1 mm, führt dies mit den vorab genannten Bestimmungsgleichungen zu einer Wellenausbreitungsgeschwindigkeit $c$ = 40.8 m/s. Der Elastizitätsmodul des elastischen Wandungsmaterials bestimmt dominant diese Phasengeschwindigkeit c der Druckwelle.

Die Ausbreitung einer eindimensionalen Welle kann mit der partiellen Differentialgleichung 2. Ordnung beschrieben werden. Sie wird Wellengleichung bzw. d'Alembert-Gleichung genannt.

$$\frac{\partial^2 p}{\partial t^2} = c^2 \frac{\partial^2 p}{\partial x^2}$$

Die Lösung der hyperbolischen Wellengleichung wird mittels numerischer Verfahren wie z.B. dem Charakteristikenverfahren durchgeführt.

Gemäß Abbildung 1 wird eine Fluidik Simulation beispielsweise in Anwendung des Rechenprogramms Matlab/Simulink der Firma Mathworks durchgeführt.

Die Fig. 6 zeigt die hydraulische Anordnung der einzelnen Elemente in einem Modell gemäß dem Stand der Technik. Neben den bereits bekannten und oben beschriebenen Bezugszeichen und deren zugeordneten Komponenten, sind ein in der Kassette 20 angeordneter Teil 26 der Aspirationsleitung (ASP-Leitung), ein Druckmesser 27 für den Irrigationszweig 13, ein strömungstechnisch einen Widerstand des Irrigationsteils im Handstück 6 charakterisierendes Element 28, ein die Hohlnadel 10 strömungstechnisch als Widerstand charakterisierendes Element 29 und ein weiterer Druckmesser 30 modellhaft gezeigt.

Des Weiteren sind in Fig. 6 gemäß der Modellierung ein strömungstechnisch die Kapazität des Auges II charakterisierendes Element 31 (EYE C) und ein Strömungswiderstand 32 (EYE R), sowie ein den Durchbruch eines Linsenpartikels im Handstück 6 charakterisierender Schalter 33 gezeigt. Darüber hinaus sind ein elastischer Irrigationsschlauch 140 und ein weitere elastischer Schlauch (BSS-Schlauch) zwischen dem Behälter 15 und der Kassette 20 durch das Element 141 modellhaft symbolisiert.

Die für die Ausbreitung der Druckwellen relevanten elastischen Leitungen sind mit dem Leitungs-Symbol dargestellt. Das Rechenprogramm Simulink diskretisiert die elastische Leitung in mehrere kleine Segmente die jeweils aus einer einzelnen Elastizität, Kapazität sowie mit einem Widerstand dargestellt sind, wie dies in Fig. 7 gezeigt ist. Das Übertragungsverhalten im Zeitbereich wird mittels des Charakteristikenverfahrens berechnet.

Die Pumpe 2 im Simulationsmodell generiert einen nichtkontinuierlichen Flow Q(t). Es wird von einer Peristaltikpumpe ausgegangen, die bei einer Maximaldrehzahl n = 600 1/min einen maximalen Flow Q von 60 ml/min generieren kann. Das Pumpenrad wird mit sieben Rollen 3 angenommen. Die maximale Anregungsfrequenz der sich drehenden Pumpenrollen 3 beträgt somit:

$$f_{\max} = \frac{600}{60\,\sec} \cdot 7 = 70\,Hz$$

Die Peristaltikpumpe 2 wird als Randbedingung im Simulationsnetzwerk angekoppelt und fördert aus dem Aspirationsbereich einen Volumenstrom nach folgendem Gesetz:

$$Q(t) = \frac{n}{600} \cdot 60\,ml \cdot (1 + 1/4 \cdot \sin(7 \cdot \pi/30 \cdot n \cdot t))$$

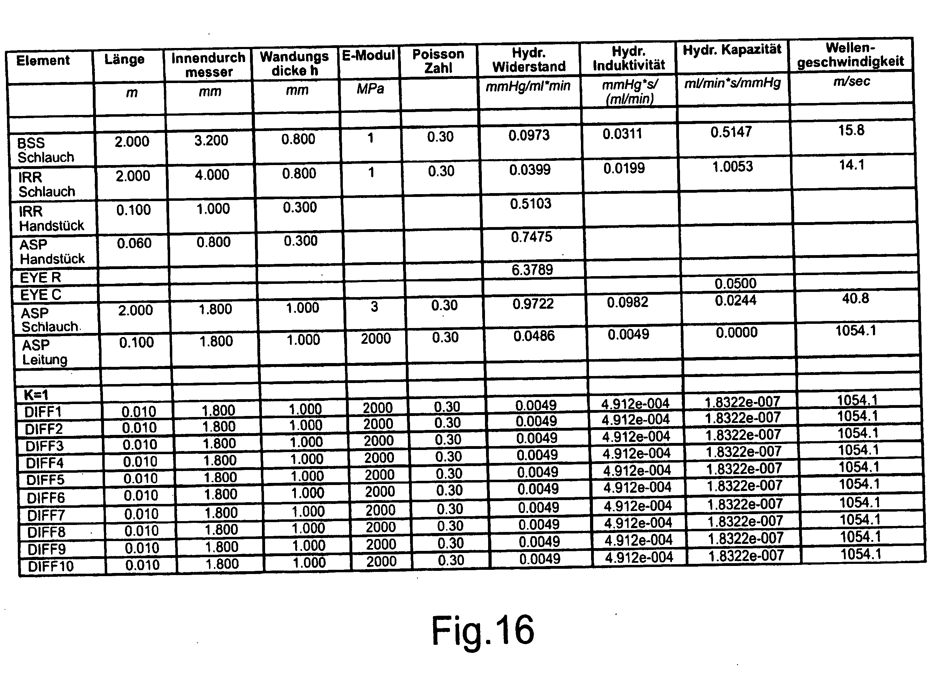

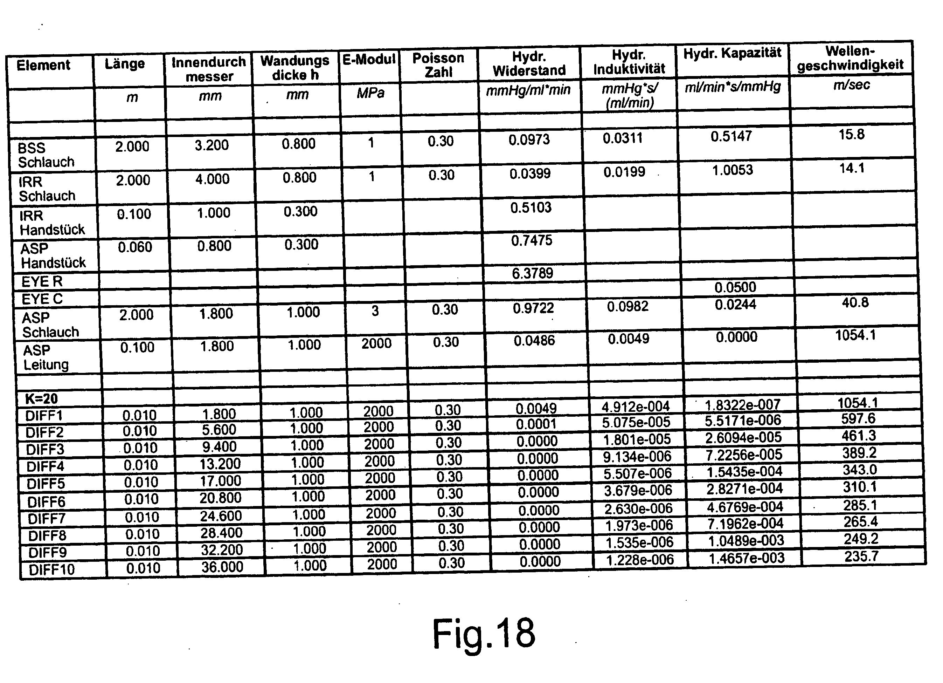

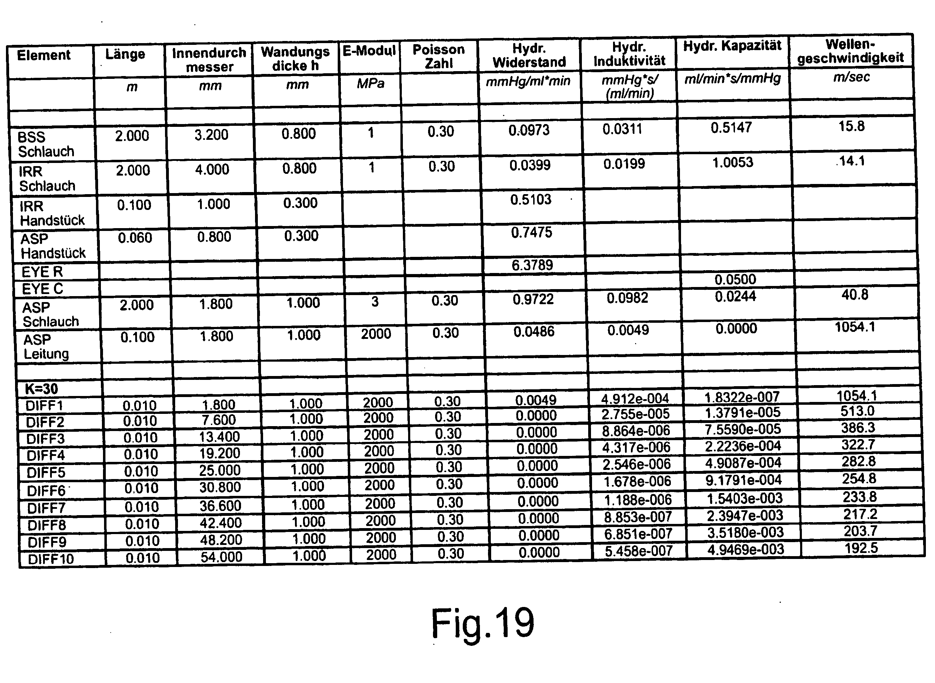

In den Fig. 16 bis 19 sind Tabellen gezeigt, die Eingangsdaten für die hydraulische Simulation darlegen. Die charakteristischen Kenngrößen und die hydraulischen Eigenschaften sind dabei pro Element dargestellt. Das System wird wie folgt durchgestimmt: Die Peristaltikpumpe 2 wird in einem Zeitraum von *t*=0 bis 240 Sekunden linear von 0 bis 600 1/min entsprechend 0 bis 60 ml/min im Flow gesteigert und anschließend im Endwert konstant gelassen. Analog verläuft die Flowpulsanregung von 0 bis 70 Hz.

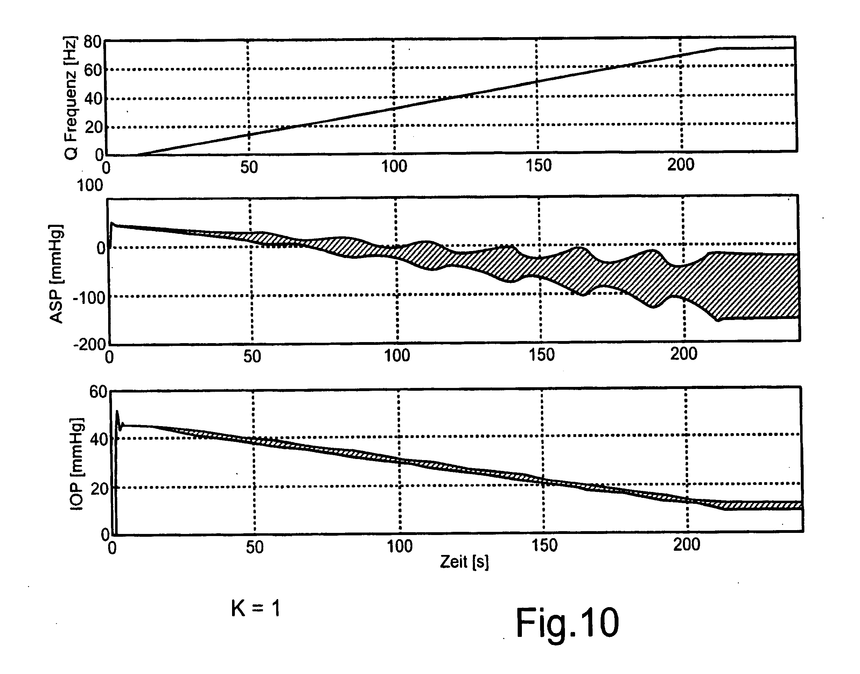

In Fig. 10 zeigt die obere Kurve die kontinuierlich konstant ansteigende Pumpendrehzahl beziehungsweise die Frequenz der Volumenstrompulse Q(t) bis zur Maximaldrehzahl von n = 600 1/min, entsprechend f = 10*7 = 70 Hz. Die darunter dargestellte Druckpulsationskurve (ASP) zeigt die Druckschwingungen an der Pumpenseite im Aspirationszweig. Es sind bei bestimmten Frequenzen deutliche Resonanzen sichtbar, an denen sich die Druckpulsationen deutlich stärker herausbilden. Ursache dafür ist, dass sich die Druckwellen in dem elastischen Leitungssystem mit einer Geschwindigkeit von ca. 40,8 m/s ausbreiten und sich auf Grund der Randbedingungen bei bestimmten Frequenzen durch das reflektierende Verhalten eine stehende Welle bilden kann. Bei einem Schwingungsbauch und einem Schwingungsknoten einer elastischen Aspirationsleitung (*1*=2 m, *c*= 40.8 m/s) lässt sich die erste Grundfrequenz nach folgenden Gesetzen bestimmen:

$$\lambda_0 = 4 \cdot l$$

$$f_0 = c/\lambda_0$$

Die nächsten höheren Resonanzfrequenzen der stehenden Wellen bestimmen sich weiter durch die Multiplikation der Grundresonanzfrequenz $f_0$ mit den aufsteigenden ungeraden Zahlen zu:

$$\lambda_1 = 4/3 \cdot l$$

$$f_1 = c/\lambda_1$$

und

$$\lambda_2 = 4/5 \cdot l$$

$$f_2 = c/\lambda_2$$

usw.

Die Resonanzfrequenzen lassen sich mit 1 = 2 m, $c$ = 40.8 m/s somit bestimmen zu:

$f_0$ = 5.1 Hz, $f_1$ = 15.3 Hz, $f_2$ = 25.5 Hz usw.. Diese Resonanzfrequenzen der stehenden Wellen des elastischen Leitungssystems sind im Aspirationsdruckverlauf in Fig. 10 deutlich zu sehen. Die kontinuierliche Durchstimmung der Anregungsfrequenz der Peristaltikpumpe 2 zeigt deutlich das Ansprechverhalten der elastischen Aspirationsleitung hinsichtlich der abgeschätzten, resonanten stehenden Eigenschwingungen. Das dritte Diagramm in Fig. 10 zeigt die aus den Druckpulsationen in der Aspirationsleitung an das Auge II übertragenen Druckschwingungen (Augeninnendruck IOP) in Abhängigkeit von der Zeit.

Die aus den Grundabschätzungen hergeleiteten Resonanzfrequenzen sind in Fig. 10 deutlich zu sehen.

Durch die Erfindung ist es gewährleistet, die Druckpulsationen im Auge, die durch die stehenden Wellen im Aspirationsleitungssystem angeregt werden, so zu dämpfen bzw. eine Impedanztransformation durchzuführen, dass keine zusätzliche Kapazität im Aspirationszweig eingebracht wird und keine Verminderung des Leitungsquerschnittes innerhalb des Aspirationszweigs zur Anwendung kommen muss.

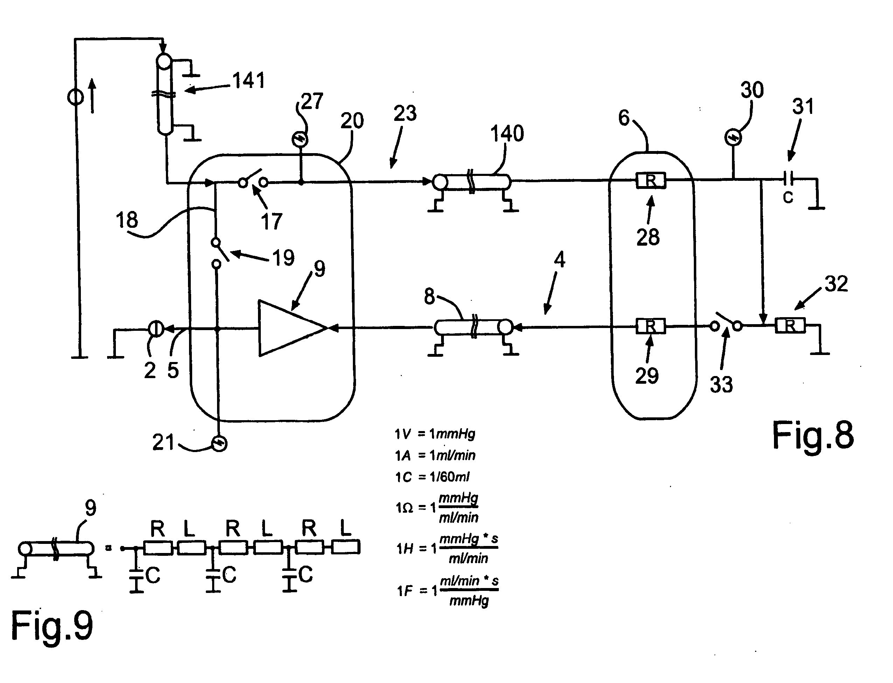

Dies erfolgt gemäß der Erfindung mit der Vorrichtung, welche stromauf der Pumpe 2 und stromab des Handstücks 6 eine Diffusoranordnung 9 umfasst. Diese Diffusoranordnung 9 ist so ausgebildet, dass sie die Eigenfrequenzbereiche der stehenden Wellen des Aspirationszweigs 4 so transformiert, dass keine Druckpulsationsanregung mehr in die Augenkammer gelangt. Eine für die Simulationsrechnung zugrunde gelegte Modellierung des Teilsystems des chirurgischen Systems I ist in Fig. 8 gezeigt. Im Unterschied zur Fig. 6 ist hier der Teil 26 durch die Diffusoranordnung 9 ersetzt. In Fig. 9 ist angedeutet, dass für die strömungstechnische Modellierung für die Diffusoranordnung 9 ebenfalls ein Ersatzschaltbild aus Kapazitäten, Induktivitäten und Widerständen herangezogen werden kann, welches individuell für die Simulation ausgestaltet werden kann.

Das oder die Diffusorelemente einer Diffusoranordnung 9 besitzen gemäß oben angeführter Erläuterung ein kleines Innenmaß D1 und ein großes Innenmaß D2. Das Verhältnis K der Innenmaße wird als Öffnungsfaktor bezeichnet:

$$K = D2/D1$$

Die Länge L der Diffusoranordnung 9 soll im Ausführungsbeispiel etwa 5 % der Länge des elastischen Aspirationsschlauches (ASP-Schlauch) sein, also ca. $L$ = 100 mm bei einem Aspirationsschlauch mit einer Länge von 2 m betragen.

Gemäß dem Modell nach Fig. 8 wird eine Simulation mit einem Diffusormodell durchgeführt. Der Diffusor ist in einer Diskretisierung nach Fig. 4f mit äquidistanten Durchmesserstufen im Modell eingebunden und umfasst zehn Teilsegmente DIFF1 bis DIFF10.

Die Diffusoranordnung 9 hat die identische Wandstärke h und Länge und das gleiche Kunststoffmaterial wie in Fig. 6 der Teil 26 der zylindrischen Aspirationsleitung in der Kassette 20 im Modell nach dem Stand der Technik. Das Modell nach dem Stand der Technik besitzt somit ein Rohr mit einem Öffnungsfaktor K=1, wenn die Definition oben für einen Diffusor zugrunde gelegt werden soll. Dabei wird der identische Simulationsverlauf angenommen wie beim Modell nach dem Stand der Technik, wobei die Parameter für die Simulation des Stands der Technik-Modells durch die Tabelle gemäß Fig. 16 gezeigt sind.

Für die Bestimmung der optimalen Ausgestaltung der Diffusoranordnung 9 wird insbesondere der Öffnungsfaktor K des Diffusors optimiert.

Als Werkstoff für die Aspirationsleitung in der Kassette 20 bzw. dem Diffusor wird dabei Polycarbonat betrachtet, das als kostengünstiger Stritzgußwerkstoff verwendet werden kann. Der E-Modul wird z.B. mit 2 GPa angenommen.

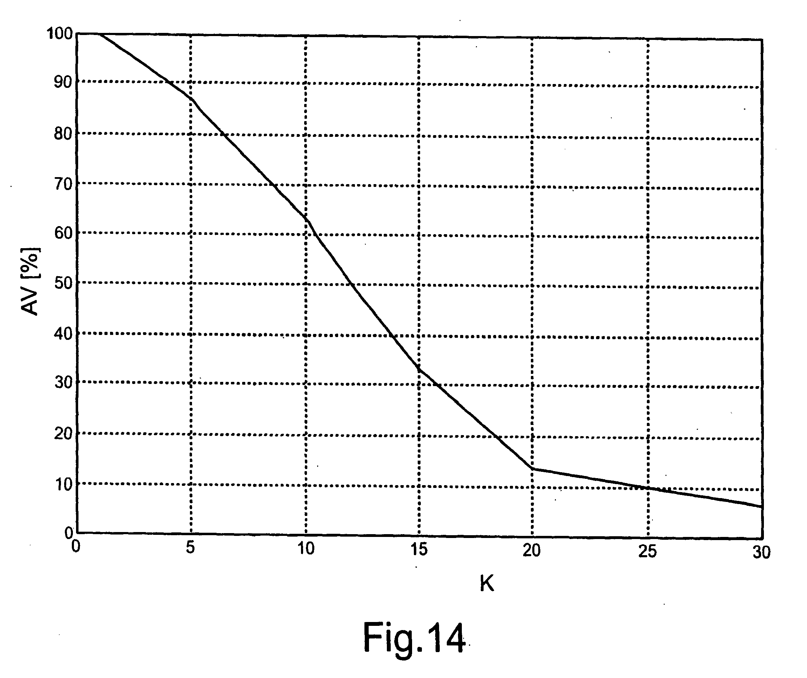

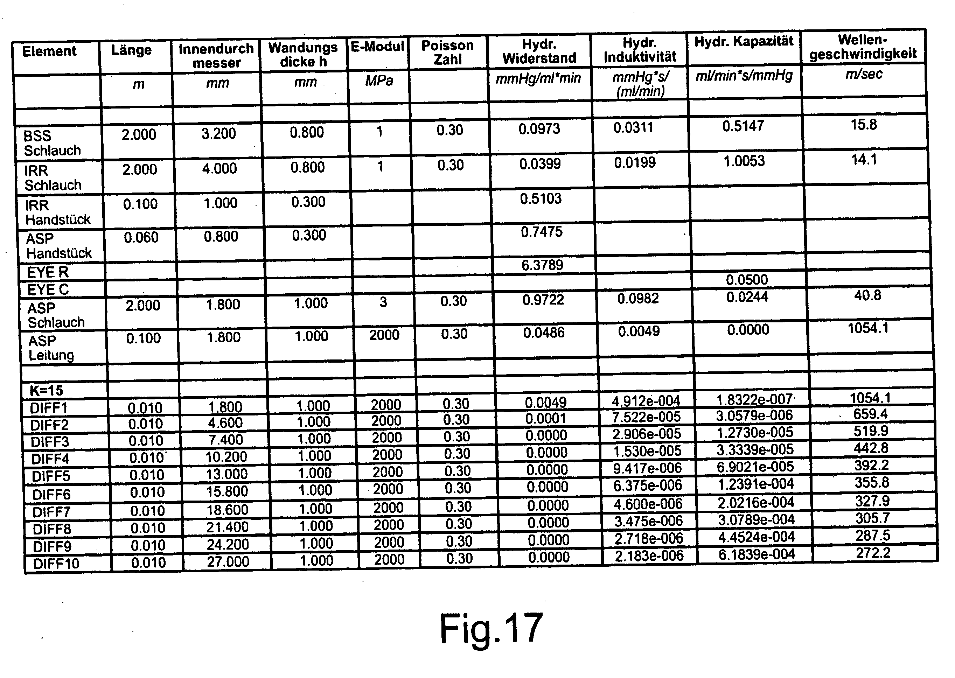

Es zeigt sich, dass gemäß Fig. 14 bei einem Öffnungsfaktor von K > 1 das Übertragungs- und Resonanzverhalten im Aspirationszweig 4 deutlich verändert wird. In Fig. 14 ist das Verhältnis AV der Schwingungsamplituden bei dem jeweiligem Wert K > 1 bezogen auf die Schwingungsamplitude bei K=1 in Prozent dargestellt, wobei

$$ AV = \frac{A_{K=x}}{A_{K=1}} $$

gilt. Ab einem Öffnungsfaktor von K > 10 bis K=30 tritt eine deutliche Reduzierung der Schwingungsamplituden ein. Die Tabellen in Fig. 17 bis 19 zeigen die für die Fälle K=15, K=20, K=30 der Simulation zu Grunde liegenden Parameter des optimierten Diffusorelements mit den jeweiligen Diffusorinkrementen DIFF 1 bis DIFF10 gemäß Fig. 4f.

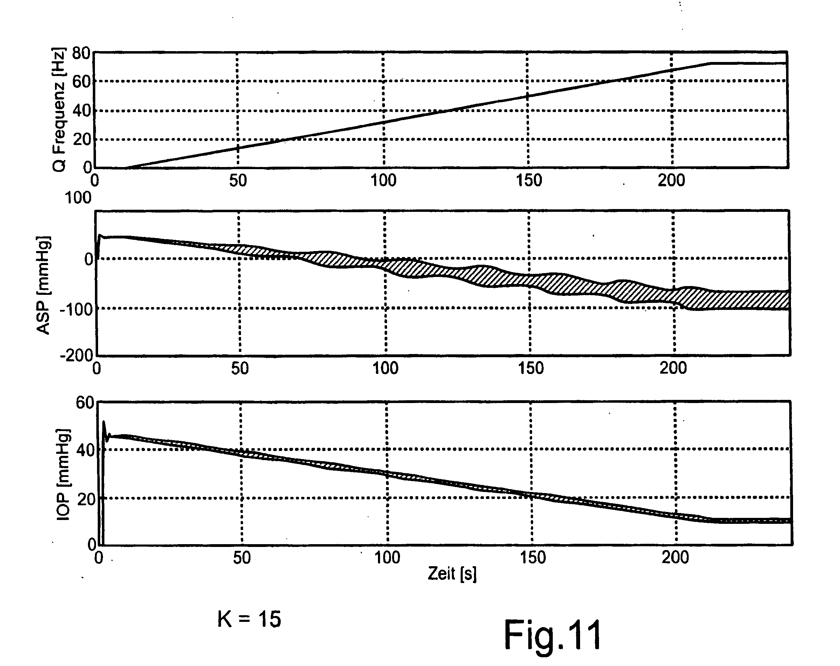

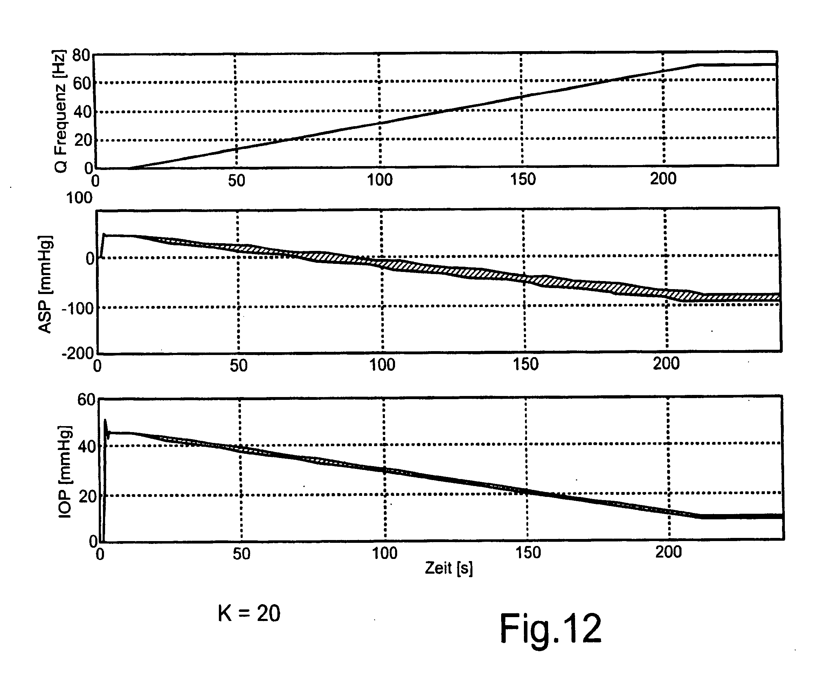

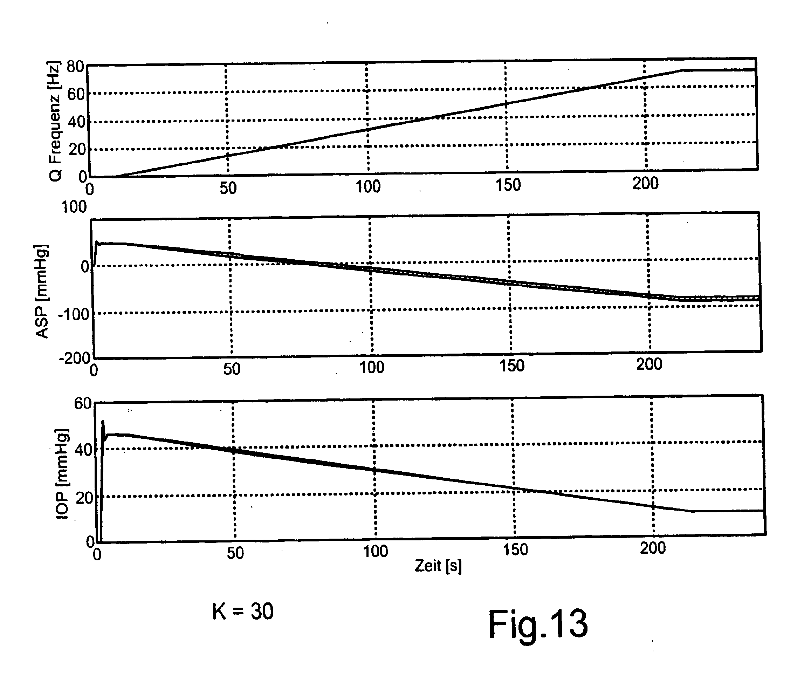

Die Diagramme in den Fig. 10 bis 13 zeigen den Ausgangssimulationsverlauf K=1, K=15, K=20 und K=30. Während die Eigenfrequenzen nahezu unverändert bleiben, vermindern sich mit zunehmendem K die Amplituden des Intraokulardruckes IOP wie auch des Aspirationsdruckes. Im Auge II sind bei hohem K fast keine Pulsationen mehr zu bemerken.

Das kleinere Innenmaß D1 ist bevorzugt gleich dem Innenmaß der elastischen Aspirationsschlauchs.

Der Einsatz von Diffusorelementen im Aspirationszweig 4 führt bei entsprechender Auslegung mit einem Simulationsprogramm wie Matlab/Simulink oder z.B. 3D- Multi-Physik Tools, wie Comsol, zu einer optimierten Auslegung der Diffusoranordnung 9.

Die Pulsübertragungsfunktion des Aspirationszweigs 4 kann in Verbindung mit dem Einsatz einer Peristaltikpumpe 2 und einem elastischen Aspirationsschlauchsystem derart ausgelegt werden, dass die Druckpulsationen einer Peristaltikpumpe 2 deutlich gesenkt werden können, ohne dass zusätzliche Kapazitäten im Aspirationsbereich eingebracht werden müssen oder der innere Querschnitt der Saugleitung deutlich vermindert werden muss.

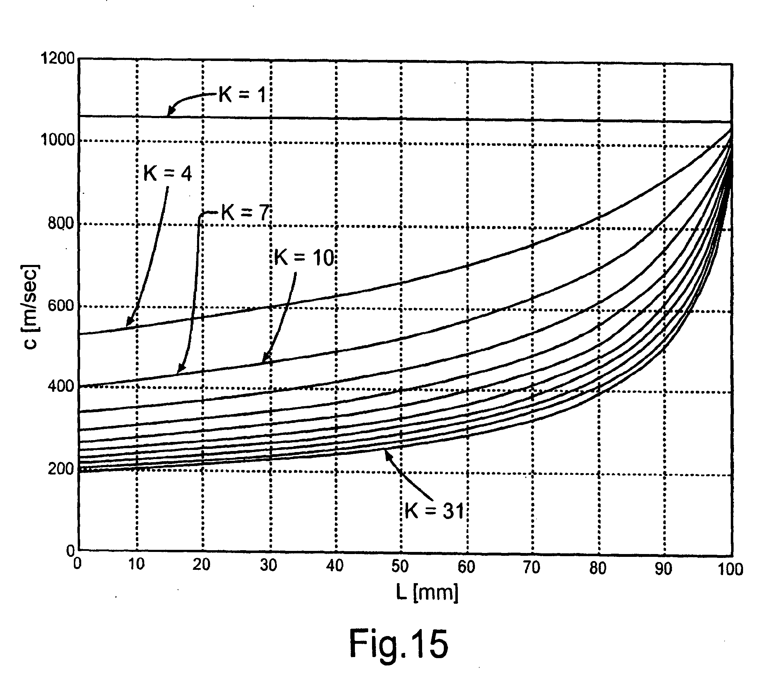

Fig. 15 zeigt den Verlauf der Phasengeschwindigkeit c entlang der Längsachse einer Diffusoranordnung 9 über deren Länge L, wobei das Fluid analog beispielsweise zu den Anordnungen in den Fig. 2 und 8 von der rechten Seite zugeführt wird und an der linken Seite die Diffusoranordnung 9 in Richtung der Pumpe 2 verlässt. Der Wert O im Fig. 15 liegt somit am größeren Innenmaß D2 und der Wert 100 am kleineren Innenmaß D1.

Für K=1 ist keine Veränderung der Phasengeschwindigkeit c entlang der Längsachse feststellbar. Bei höheren Werten von K reduziert sich die Phasengeschwindigkeit c ausgehend vom kleinen Innenmaß D1 zum großen Innenmaß D2 entlang der Längsachse mit zunehmender Diffusorlänge L. Fig. 15 zeigt Kennlinien für verschiedene K-Werte von K=1 bis K=31.

Die genannten Optimierungseigenschaften Querschnitt, Mensur, Länge sowie besonders der Öffnungsfaktor K werden vorzugsweise mit einem 3D Multiphysiks Tool wie z.B Comsol Multiphysiks für die entsprechende Applikation optimiert.

In der oben erläuterten Simulation zur Bestimmung der Ausgestaltung einer Diffusoranordnung 9 wurde gemäß den Tabellen in den Fig. 17 bis 19 lediglich die Größe K als Variable herangezogen und die weiteren genannten Größen, wie BSS-Schlauch etc. als feste Parameter mit den beispielhaft genannten Werten zugrunde gelegt. Da die Größe K den wesentlichen Einfluss bezüglich einer Dämpfung der Amplitude der Druckwelle aufweist, ist deren Optimierung im Vordergrund. Es kann aber auch vorgesehen sein, dass für eine weitere Verbesserung auch die Größen, BSS-Schlauch und/oder IRR-Schlauch und/oder IRR-Handstück und/oder ASP-Handstück und/oder EYE R und/oder EYE C und/oder ASP-Schlauch und/oder ASP-Leitung mit anderweitigen festen Werten zugrunde gelegt werden oder aber sogar selbst als Variable im Simulationslauf definiert werden. Allerdings ist anzumerken, dass diese genannten Größen im Vergleich zur Größe K einen deutlich geringeren Einfluss, unter spezifischen Bedingungen auch einen vernachlässigbaren Einfluss, auf die Dämpfung der Amplitude der Druckwellen haben.

**Patentansprüche**

1. Vorrichtung zur Reduzierung von Druckschwankungen eines in einem Aspirationszweig (4) für ein chirurgisches System (I) strömenden Fluids, mit dem Aspirationszweig (4) und mit einer Pumpe (2), welche die Druckschwankungen durch eine im aktiven Betrieb nicht-kontinuierliche Förderung des Fluids erzeugt, **dadurch gekennzeichnet, dass** eine Diffusoranordnung (9) im Aspirationszweig (4) in Strömungsrichtung des Fluids vor der Pumpe (2) angeordnet ist, und welche ein Verhältnis K zwischen ihrem maximalen Innenmaß (D2) und ihrem minimalen Innenmaß (D1) aufweist, welches größer oder gleich 2 ist.

**2.** Vorrichtung nach Anspruch 1, bei welcher das Verhältnis K der Diffusoranordnung (9) zwischen ihrem maximalen Innenmaß (D2) und ihrem minimalen Innenmaß (D1) größer oder gleich 10 ist.

**3.** Vorrichtung nach Anspruch 1, bei welcher die Diffusoranordnung (9) lösbar im Aspirationszweig (4) angeordnet ist.

**4.** Vorrichtung nach Anspruch 1, bei welcher das minimale Innenmaß (D1) des mit Fluid durchströmten Bereichs der Diffusoranordnung (9) größer oder gleich dem minimalen Innenmaß (D1) einer vorderen Öffnung einer Hohlnadel (10) eines chirurgischen Handstücks (6) ist.

**5.** Vorrichtung nach Anspruch 4, bei welcher das minimale Innenmaß (D1) der Diffusoranordnung (9) zwischen 0,8 mm und 2,5 mm, insbesondere zwischen 0,8 mm und 1,5 mm, ist.

**6.** Vorrichtung nach Anspruch 4, bei welcher das maximale Innenmaß (D2) des mit Fluid durchströmten Bereichs der Diffusoranordnung (9) zwischen 1,2 mm und 25 mm, insbesondere zwischen 1,5 und 15 mm ist.

**7.** Vorrichtung nach Anspruch 1, bei welcher die Diffusoranordnung (9) mit einem elastischen Aspirationsschlauch (8) des Aspirationszweigs (4) verbunden ist und die Länge der Diffusoranordnung (9) kleiner 15% der Länge des Aspirationsschlauchs (8), insbesondere kleiner oder gleich 5% der Länge des Aspirationsschlauchs (8), beträgt.

**8.** Vorrichtung nach Anspruch 1, bei welcher die Diffusoranordnung (9) eine Länge zwischen 10 mm und 400 mm, insbesondere zwischen 90 mm und 300 mm, vorzugsweise kleiner oder gleich 200 mm, aufweist.

**9.** Vorrichtung nach Anspruch 1, bei welcher die Diffusoranordnung (9) in einer Fluidik-Kassette (20) angeordnet ist.

**10.** Vorrichtung nach Anspruch 1, bei welcher die Diffusoranordnung (9) ein Verhältnis K zwischen ihrem maximalen Innenmaß (D2) und ihrem minimalen Innenmaß (D1) aufweist, wobei das minimale (D1) und das maximale Innenmaß (D2) an den Endöffnungen der Diffusoranordnung (9) ausgebildet sind.

**11.** Vorrichtung nach Anspruch 1, bei welcher zumindest ein Diffusor (22, 23) der Diffusoranordnung (9) über seine Länge ein sich ausgehend von einem minimalen Innenmaß (D1) bis zu einem maximalen Innenmaß (D2) stetig vergrößerndes Innenmaß aufweist.

**12.** Vorrichtung nach Anspruch 1, bei welcher eine Innenseite (24, 25) eines Diffusors (22, 23) der Diffusoranordnung (9) in Längsrichtung des Diffusors (22, 23) zumindest bereichsweise gekrümmt ausgebildet ist.

**13.** Vorrichtung nach Anspruch 1, bei welcher eine Innenseite (24, 25) eines Diffusors (22, 23) der Diffusoranordnung (9) in Längsrichtung des Diffusors (22, 23) zumindest bereichsweise gestuft ausgebildet ist.

**14.** Chirurgisches System, insbesondere ophthalmisches mikrochirurgisches System zur Phako-Chirurgie, mit einer im aktiven Betrieb nicht-kontinuierlich fördernden Pumpe (2), welche einem Aspirationszweig (4) des Systems (I) zugeordnet ist, einem chirurgischen Handstück (6), welches mit dem Aspirationszweig (4) verbunden ist, und einer Vorrichtung nach eine der Ansprüche 1-13.

**15.** Chirurgisches System nach Anspruch 14, bei welchem die Diffusoranordnung (9) beabstandet zur mit der Aspirationsleitung verbundenen Pumpe (2) und beabstandet zu einem mit einem elastischen Aspirationsschlauch (8) des Aspirationszweigs (4) verbundenen chirurgischen Handstück (6) zwischen der Pumpe (2) und dem Handstück (6) angeordnet ist.

## Claims

**1.** A device for reducing pressure variations of a fluid flowing in an aspiration branch (4) for a surgical system (I), comprising the aspiration branch (4) and a pump (2), which generates the pressure variations by a discontinuous delivery of the fluid in an active operational state, **characterized in that** a diffuser arrangement (9) is arranged in the aspiration branch (4) in the flow direction of the fluid before the pump (2), and which has a ratio K between its maximum internal dimension (D2) and its minimum internal dimension (D1), which is greater than or equal to 2.

**2.** The device as claimed in claim 1, in which the ratio K of the diffuser arrangement (9) between its maximum internal dimension (D2) and its minimum internal dimension (D1) is greater than or equal to 10.

3.  The device as claimed in claim 1, in which the diffuser arrangement (9) is releasably arranged in the aspiration branch (4).

4.  The device as claimed in claim 1, in which the minimum internal dimension (D1) of the region of the diffuser arrangement (9) through which fluid flows is greater than or equal to the minimum internal dimension (D1) of a front opening of a hollow needle (10) of a surgical handpiece (6).

5.  The device as claimed in claim 4, in which the minimum internal dimension (D1) of the diffuser arrangement (9) lies between 0.8 mm and 2.5 mm, in particular between 0.8 mm and 1.5 mm.

6.  The device as claimed in claim 4, in which a maximum internal dimension (D2) of the region of the diffuser arrangement (9) through which fluid flows lies between 1.2 mm and 25 mm, in particular between 1.5 mm and 15 mm.

7.  The device as claimed in claim 1, in which the diffuser arrangement (9) is connected to an elastic aspiration tube (8) of the aspiration branch (4) and the length of the diffuser arrangement (9) is less than 15% of the length of the aspiration tube (8), in particular less than or equal to 5% of the length of the aspiration tube (8).

8.  The device as claimed in claim 1, in which the diffuser arrangement (9) has a length of between 10 mm and 400 mm, in particular between 90 mm and 300 mm, preferably less than or equal to 200 mm.

9.  The device as claimed in claim 1, in which the diffuser arrangement (9) is arranged in a fluidics cassette (20).

10. The device as claimed in claim 1, in which the diffuser arrangement (9) has a ratio K between its maximum internal dimension (D2) and its minimum internal dimension (D1), wherein the minimum internal dimension (D1) and the maximum internal dimension (D2) are formed at the end openings of the diffuser arrangement (9).

11. The device as claimed in claim 1, in which at least one diffuser (22, 23) in the diffuser arrangement (9) has an internal dimension that continuously increases over its length, starting from a minimum internal dimension (D1) up to a maximum internal dimension (D2).

12. The device as claimed in claim 1, in which an inner side (24, 25) of a diffuser (22, 23) in the diffuser arrangement (9) has at least in part a curved design in the longitudinal direction of the diffuser (22, 23).

13. The device as claimed in claim 1, in which an inner side (24, 25) of a diffuser (22, 23) in the diffuser arrangement (9) has at least in part a stepped design in the longitudinal direction of the diffuser (22, 23).

14. A surgical system, in particular an ophthalmic microsurgical system for lens surgery, comprising a pump (2) delivering in a discontinuous fashion in an active operational state, the pump being associated with an aspiration branch (4) of the system (I), a surgical handpiece (6) connected to the aspiration branch (4), and a device according to any one of claims 1 to 13.

15. The surgical system as claimed in claim 14, in which the diffuser arrangement (9) is arranged between the pump (2) and a surgical handpiece (6), at a distance from the pump (2) connected with the aspiration branch, and at a distance from said handpiece (6) connected by an elastic aspiration tube (8) of the aspiration branch (4).

**Revendications**

1.  Mécanisme, destiné à réduire des variations de pression dans une branche d'aspiration (4) pour un système chirurgical (I) d'un fluide qui s'écoule, avec la branche d'aspiration (4) et avec une pompe (2), qui produit les variations de pression par le biais d'un acheminement du fluide non continu en mode actif, **caractérisé en ce qu'**un agencement du diffuseur (9) est disposé dans la branche d'aspiration (4), dans le sens d'écoulement du fluide, avant la pompe (2) et présente un rapport K entre sa dimension intérieure maximale (D2) et sa dimension intérieure minimale (D1), lequel est supérieur ou égal à 2.

2.  Mécanisme suivant la revendication 1, pour lequel le rapport K de l'agencement du diffuseur (9), entre sa dimension intérieure maximale (D2) et sa dimension intérieure minimale (D1), est supérieur ou égal à 10.

3. Mécanisme suivant la revendication 1, pour lequel l'agencement du diffuseur (9) est disposé de manière amovible dans la branche d'aspiration (4).

4. Mécanisme suivant la revendication 1, pour lequel la dimension intérieure minimale (D1) de la zone, parcourue par le fluide, de l'agencement du diffuseur (9) est supérieure ou égale à la dimension intérieure minimale (D1) d'une ouverture antérieure d'une canule creuse (10) d'une pièce à main chirurgicale (6).

5. Mécanisme suivant la revendication 4, pour lequel la dimension intérieure minimale (D1) de l'agencement du diffuseur (9) se situe entre 0,8 mm et 2,5 mm, en particulier entre 0,8 mm et 1,5 mm.

6. Mécanisme suivant la revendication 4, pour lequel la dimension intérieure maximale (D2) de la zone, parcourue par le fluide, de l'agencement du diffuseur (9) se situe entre 1,2 mm et 25 mm, en particulier entre 1,5 et 15 mm.

7. Mécanisme suivant la revendication 1, pour lequel l'agencement du diffuseur (9) est relié à un tuyau d'aspiration élastique (8) de la branche d'aspiration (4) et la longueur de l'agencement du diffuseur (9) est inférieure de 15 % à la longueur du tuyau d'aspiration (8), inférieure ou égale, en particulier, de 5 % à la longueur du tuyau d'aspiration (8).

8. Mécanisme suivant la revendication 1, pour lequel l'agencement du diffuseur (9) présente une longueur entre 10 mm et 400 mm, en particulier entre 90 mm et 300 mm, de préférence inférieure ou égale à 200 mm.

9. Mécanisme suivant la revendication 1, pour lequel l'agencement du diffuseur (9) est disposé dans une cartouche de fluide (20).

10. Mécanisme suivant la revendication 1, pour lequel l'agencement du diffuseur (9) présente un rapport K entre sa dimension intérieure maximale (D2) et sa dimension intérieure minimale (D1), moyennant quoi la dimension intérieure minimale (D1) et maximale (D2) sont formées aux ouvertures d'extrémité de l'agencement du diffuseur (9).

11. Mécanisme suivant la revendication 1, pour lequel au moins un diffuseur (22, 23) de l'agencement du diffuseur (9) présente, sur sa longueur, une dimension intérieure, croissant en continu, en partant d'une dimension intérieure minimale (D1) jusqu'à une dimension intérieure maximale (D2).

12. Mécanisme suivant la revendication 1, pour lequel une face interne (24, 25) d'un diffuseur (22, 23) de l'agencement du diffuseur (9) est formée, dans le sens longitudinal du diffuseur (22, 23), au moins par endroits, de manière courbée.

13. Mécanisme suivant la revendication 1, pour lequel une face interne (24, 25) d'un diffuseur (22, 23) de l'agencement du diffuseur (9) est formée, dans le sens longitudinal du diffuseur (22, 23), au moins par endroits, de manière étagée.

14. Système chirurgical, en particulier système microchirurgical ophtalmique pour la phaco-chirurgie, avec une pompe (2), qui achemine de manière non continue en mode actif, laquelle est affectée à une branche d'aspiration (4) du système (I), une pièce à main chirurgicale (6), laquelle est reliée à la branche d'aspiration (4) et un mécanisme suivant l'une des revendications 1 à 13.

15. Système chirurgical suivant la revendication 14, pour lequel l'agencement du diffuseur (9) est agencé, entre la pompe (2) et la pièce à main (6), écarté par rapport à la pompe (2), reliée à la conduite d'aspiration et écarté par rapport à une pièce à main chirurgicale (6), reliée à un tuyau d'aspiration élastique (8) de la branche d'aspiration (4).

Fig.1

Fig.2

Fig.3a

Fig.3b

Fig.3c

Fig.3d

Fig.3e

Fig.3f

Fig.3g

Fig.3h

Fig.3i

**Fig.4a**

**Fig.4b**

**Fig.4c**

**Fig.4d**

**Fig.4e**

**Fig.4f**

D2 D1
9

**Fig.5a**      **Fig.5b**      **Fig.5c**

9        9        9

**Fig.5d**      **Fig.5e**      **Fig.5f**

9        9        9

**Fig.5g**      **Fig.5h**      **Fig.5i**

9        9

**Fig.5j**      **Fig.5k**

141

27  20  13  140  6  30  31

18

17

19

4

2  26  8  28

21  29  33

**Fig.6**

$1V = 1mmHg$

$1A = 1ml/min$

$1C = 1/60ml$

$1\Omega = 1\dfrac{mmHg}{ml/min}$

$1H = 1\dfrac{mmHg \cdot s}{ml/min}$

$1F = 1\dfrac{ml/min \cdot s}{mmHg}$

26

R  L  R  L  R  L

C  C  C

**Fig.7**

EP 2 164 540 B1

Fig.8

Fig.9

K = 1

Fig.10

Fig.11

K = 15

K = 20

Fig.12

EP 2 164 540 B1

K = 30

Fig.13

EP 2 164 540 B1

Fig.14

Fig.15

| Element | Länge | Innendurch messer | Wandungs dicke h | E-Modul | Poisson Zahl | Hydr. Widerstand | Hydr. Induktivität | Hydr. Kapazität | Wellen- geschwindigkeit |
|---|---|---|---|---|---|---|---|---|---|
| | m | mm | mm | MPa | | mmHg/ml*min | mmHg*s/ (ml/min) | ml/min*s/mmHg | m/sec |
| | | | | | | | | | |
| BSS Schlauch | 2.000 | 3.200 | 0.800 | 1 | 0.30 | 0.0973 | 0.0311 | 0.5147 | 15.8 |
| IRR Schlauch | 2.000 | 4.000 | 0.800 | 1 | 0.30 | 0.0399 | 0.0199 | 1.0053 | 14.1 |
| IRR Handstück | 0.100 | 1.000 | 0.300 | | | 0.5103 | | | |
| ASP Handstück | 0.060 | 0.800 | 0.300 | | | 0.7475 | | | |
| EYE R | | | | | | 6.3789 | | | |
| EYE C | | | | | | | | 0.0500 | |
| ASP Schlauch | 2.000 | 1.800 | 1.000 | 3 | 0.30 | 0.9722 | 0.0982 | 0.0244 | 40.8 |
| ASP Leitung | 0.100 | 1.800 | 1.000 | 2000 | 0.30 | 0.0486 | 0.0049 | 0.0000 | 1054.1 |
| | | | | | | | | | |
| K=1 | | | | | | | | | |
| DIFF1 | 0.010 | 1.800 | 1.000 | 2000 | 0.30 | 0.0049 | 4.912e-004 | 1.8322e-007 | 1054.1 |
| DIFF2 | 0.010 | 1.800 | 1.000 | 2000 | 0.30 | 0.0049 | 4.912e-004 | 1.8322e-007 | 1054.1 |
| DIFF3 | 0.010 | 1.800 | 1.000 | 2000 | 0.30 | 0.0049 | 4.912e-004 | 1.8322e-007 | 1054.1 |
| DIFF4 | 0.010 | 1.800 | 1.000 | 2000 | 0.30 | 0.0049 | 4.912e-004 | 1.8322e-007 | 1054.1 |
| DIFF5 | 0.010 | 1.800 | 1.000 | 2000 | 0.30 | 0.0049 | 4.912e-004 | 1.8322e-007 | 1054.1 |
| DIFF6 | 0.010 | 1.800 | 1.000 | 2000 | 0.30 | 0.0049 | 4.912e-004 | 1.8322e-007 | 1054.1 |
| DIFF7 | 0.010 | 1.800 | 1.000 | 2000 | 0.30 | 0.0049 | 4.912e-004 | 1.8322e-007 | 1054.1 |
| DIFF8 | 0.010 | 1.800 | 1.000 | 2000 | 0.30 | 0.0049 | 4.912e-004 | 1.8322e-007 | 1054.1 |
| DIFF9 | 0.010 | 1.800 | 1.000 | 2000 | 0.30 | 0.0049 | 4.912e-004 | 1.8322e-007 | 1054.1 |
| DIFF10 | 0.010 | 1.800 | 1.000 | 2000 | 0.30 | 0.0049 | 4.912e-004 | 1.8322e-007 | 1054.1 |

## Fig.16

| Element | Länge | Innendurchmesser | Wandungsdicke h | E-Modul | Poisson Zahl | Hydr. Widerstand | Hydr. Induktivität | Hydr. Kapazität | Wellengeschwindigkeit |
|---|---|---|---|---|---|---|---|---|---|
| | m | mm | mm | MPa | | mmHg/ml*min | mmHg*s/ (ml/min) | ml/min*s/mmHg | m/sec |
| | | | | | | | | | |
| BSS Schlauch | 2.000 | 3.200 | 0.800 | 1 | 0.30 | 0.0973 | 0.0311 | 0.5147 | 15.8 |
| IRR Schlauch | 2.000 | 4.000 | 0.800 | 1 | 0.30 | 0.0399 | 0.0199 | 1.0053 | 14.1 |
| IRR Handstück | 0.100 | 1.000 | 0.300 | | | 0.5103 | | | |
| ASP Handstück | 0.060 | 0.800 | 0.300 | | | 0.7475 | | | |
| EYE R | | | | | | 6.3789 | | | |
| EYE C | | | | | | | | 0.0500 | |
| ASP Schlauch | 2.000 | 1.800 | 1.000 | 3 | 0.30 | 0.9722 | 0.0982 | 0.0244 | 40.8 |
| ASP Leitung | 0.100 | 1.800 | 1.000 | 2000 | 0.30 | 0.0486 | 0.0049 | 0.0000 | 1054.1 |
| | | | | | | | | | |
| K=15 | | | | | | | | | |
| DIFF1 | 0.010 | 1.800 | 1.000 | 2000 | 0.30 | 0.0049 | 4.912e-004 | 1.8322e-007 | 1054.1 |
| DIFF2 | 0.010 | 4.600 | 1.000 | 2000 | 0.30 | 0.0001 | 7.522e-005 | 3.0579e-006 | 659.4 |
| DIFF3 | 0.010 | 7.400 | 1.000 | 2000 | 0.30 | 0.0000 | 2.906e-005 | 1.2730e-005 | 519.9 |
| DIFF4 | 0.010 | 10.200 | 1.000 | 2000 | 0.30 | 0.0000 | 1.530e-005 | 3.3339e-005 | 442.8 |
| DIFF5 | 0.010 | 13.000 | 1.000 | 2000 | 0.30 | 0.0000 | 9.417e-006 | 6.9021e-005 | 392.2 |
| DIFF6 | 0.010 | 15.800 | 1.000 | 2000 | 0.30 | 0.0000 | 6.375e-006 | 1.2391e-004 | 355.8 |
| DIFF7 | 0.010 | 18.600 | 1.000 | 2000 | 0.30 | 0.0000 | 4.600e-006 | 2.0216e-004 | 327.9 |
| DIFF8 | 0.010 | 21.400 | 1.000 | 2000 | 0.30 | 0.0000 | 3.475e-006 | 3.0789e-004 | 305.7 |
| DIFF9 | 0.010 | 24.200 | 1.000 | 2000 | 0.30 | 0.0000 | 2.718e-006 | 4.4524e-004 | 287.5 |
| DIFF10 | 0.010 | 27.000 | 1.000 | 2000 | 0.30 | 0.0000 | 2.183e-006 | 6.1839e-004 | 272.2 |

## Fig.17

| Element | Länge | Innendurch messer | Wandungs dicke h | E-Modul | Poisson Zahl | Hydr. Widerstand | Hydr. Induktivität | Hydr. Kapazität | Wellen- geschwindigkeit |
|---|---|---|---|---|---|---|---|---|---|
| | m | mm | mm | MPa | | mmHg/ml*min | mmHg*s/ (ml/min) | ml/min*s/mmHg | m/sec |
| BSS Schlauch | 2.000 | 3.200 | 0.800 | 1 | 0.30 | 0.0973 | 0.0311 | 0.5147 | 15.8 |
| IRR Schlauch | 2.000 | 4.000 | 0.800 | 1 | 0.30 | 0.0399 | 0.0199 | 1.0053 | 14.1 |
| IRR Handstück | 0.100 | 1.000 | 0.300 | | | 0.5103 | | | |
| ASP Handstück | 0.060 | 0.800 | 0.300 | | | 0.7475 | | | |
| EYE R | | | | | | 6.3789 | | 0.0500 | |
| EYE C | | | | 3 | 0.30 | 0.9722 | 0.0982 | 0.0244 | 40.8 |
| ASP Schlauch | 2.000 | 1.800 | 1.000 | 2000 | 0.30 | 0.0486 | 0.0049 | 0.0000 | 1054.1 |
| ASP Leitung | 0.100 | 1.800 | 1.000 | | | | | | |
| K=20 | | | | | | | | | |
| DIFF1 | 0.010 | 1.800 | 1.000 | 2000 | 0.30 | 0.0049 | 4.912e-004 | 1.8322e-007 | 1054.1 |
| DIFF2 | 0.010 | 5.600 | 1.000 | 2000 | 0.30 | 0.0001 | 5.075e-005 | 5.5171e-006 | 597.6 |
| DIFF3 | 0.010 | 9.400 | 1.000 | 2000 | 0.30 | 0.0000 | 1.801e-005 | 2.6094e-005 | 461.3 |
| DIFF4 | 0.010 | 13.200 | 1.000 | 2000 | 0.30 | 0.0000 | 9.134e-006 | 7.2256e-005 | 389.2 |
| DIFF5 | 0.010 | 17.000 | 1.000 | 2000 | 0.30 | 0.0000 | 5.507e-006 | 1.5435e-004 | 343.0 |
| DIFF6 | 0.010 | 20.800 | 1.000 | 2000 | 0.30 | 0.0000 | 3.679e-006 | 2.8271e-004 | 310.1 |
| DIFF7 | 0.010 | 24.600 | 1.000 | 2000 | 0.30 | 0.0000 | 2.630e-006 | 4.6769e-004 | 285.1 |
| DIFF8 | 0.010 | 28.400 | 1.000 | 2000 | 0.30 | 0.0000 | 1.973e-006 | 7.1962e-004 | 265.4 |
| DIFF9 | 0.010 | 32.200 | 1.000 | 2000 | 0.30 | 0.0000 | 1.535e-006 | 1.0489e-003 | 249.2 |
| DIFF10 | 0.010 | 36.000 | 1.000 | 2000 | 0.30 | 0.0000 | 1.228e-006 | 1.4657e-003 | 235.7 |

Fig.18

| Element | Länge | Innendurchmesser | Wandungsdicke h | E-Modul | Poisson Zahl | Hydr. Widerstand | Hydr. Induktivität | Hydr. Kapazität | Wellengeschwindigkeit |
|---|---|---|---|---|---|---|---|---|---|
| | m | mm | mm | MPa | | mmHg/ml*min | mmHg*s/(ml/min) | ml/min*s/mmHg | m/sec |
| | | | | | | | | | |
| BSS Schlauch | 2.000 | 3.200 | 0.800 | 1 | 0.30 | 0.0973 | 0.0311 | 0.5147 | 15.8 |
| IRR Schlauch | 2.000 | 4.000 | 0.800 | 1 | 0.30 | 0.0399 | 0.0199 | 1.0053 | 14.1 |
| IRR Handstück | 0.100 | 1.000 | 0.300 | | | 0.5103 | | | |
| ASP Handstück | 0.060 | 0.800 | 0.300 | | | 0.7475 | | | |
| EYE R | | | | | | 6.3789 | | | |
| EYE C | | | | | | | | 0.0500 | |
| ASP Schlauch | 2.000 | 1.800 | 1.000 | 3 | 0.30 | 0.9722 | 0.0982 | 0.0244 | 40.8 |
| ASP Leitung | 0.100 | 1.800 | 1.000 | 2000 | 0.30 | 0.0486 | 0.0049 | 0.0000 | 1054.1 |
| | | | | | | | | | |
| K=30 | | | | | | | | | |
| DIFF1 | 0.010 | 1.800 | 1.000 | 2000 | 0.30 | 0.0049 | 4.912e-004 | 1.8322e-007 | 1054.1 |
| DIFF2 | 0.010 | 7.600 | 1.000 | 2000 | 0.30 | 0.0000 | 2.755e-005 | 1.3791e-005 | 513.0 |
| DIFF3 | 0.010 | 13.400 | 1.000 | 2000 | 0.30 | 0.0000 | 8.864e-006 | 7.5590e-005 | 386.3 |
| DIFF4 | 0.010 | 19.200 | 1.000 | 2000 | 0.30 | 0.0000 | 4.317e-006 | 2.2236e-004 | 322.7 |
| DIFF5 | 0.010 | 25.000 | 1.000 | 2000 | 0.30 | 0.0000 | 2.546e-006 | 4.9087e-004 | 282.8 |
| DIFF6 | 0.010 | 30.800 | 1.000 | 2000 | 0.30 | 0.0000 | 1.678e-006 | 9.1791e-004 | 254.8 |
| DIFF7 | 0.010 | 36.600 | 1.000 | 2000 | 0.30 | 0.0000 | 1.188e-006 | 1.5403e-003 | 233.8 |
| DIFF8 | 0.010 | 42.400 | 1.000 | 2000 | 0.30 | 0.0000 | 8.853e-007 | 2.3947e-003 | 217.2 |
| DIFF9 | 0.010 | 48.200 | 1.000 | 2000 | 0.30 | 0.0000 | 6.851e-007 | 3.5180e-003 | 203.7 |
| DIFF10 | 0.010 | 54.000 | 1.000 | 2000 | 0.30 | 0.0000 | 5.458e-007 | 4.9469e-003 | 192.5 |

Fig.19

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4182385 A
- WO 8903230 A